(19) Europäisches Patentamt
European Patent Office
Office européen des brevets

(11) **EP 4 198 053 A1**

(12) **EUROPEAN PATENT APPLICATION**

(43) Date of publication:
**21.06.2023   Bulletin 2023/25**

(21) Application number: **21214769.8**

(22) Date of filing: **15.12.2021**

(51) International Patent Classification (IPC):
**C07K 14/78** (2006.01)          **A61K 38/39** (2006.01)
**A61P 43/00** (2006.01)          **A61P 9/00** (2006.01)
**C12N 15/864** (2006.01)          **C12N 15/86** (2006.01)

(52) Cooperative Patent Classification (CPC):
**C07K 14/78; A61P 9/00; A61P 43/00; C12N 15/86;**
A61K 38/00; C12N 2750/14143

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Universitätsklinikum
Hamburg-Eppendorf
20246 Hamburg (DE)**

(72) Inventors:
 • **ESCHENHAGEN, Thomas
   20257 Hamburg (DE)**

 • **BEHRENS, Charlotte
   22453 Hamnburg (DE)**
 • **ULMER, Bärbel
   8049 Zürich (CH)**
 • **FREESE, Julia
   21031 Hamburg (DE)**

(74) Representative: **Maiwald GmbH
Elisenhof
Elisenstraße 3
80335 München (DE)**

Remarks:
The complete document including Reference
Table(s) and the Sequence Listing(s) can be
downloaded from the EPO website

(54) **LMNA GENE EXPRESSION FOR TREATMENT OF LAMINOPATHIES**

(57)    The present invention relates to nucleic acid molecules encoding Lamin A, vectors, AAV and pharmaceutical compositions comprising said nucleic acid molecules for use in treatment of laminopathies.

EP 4 198 053 A1

**Description**

FIELD OF THE INVENTION

The present invention relates to nucleic acid molecules encoding Lamin A, vectors, AAV and pharmaceutical compositions comprising said nucleic acid molecules for use in treatment of laminopathies.

Background of the invention

Laminopathies are a group of rare genetic diseases caused by mutations in genes encoding for proteins associated with the nuclear envelope. Most commonly affected is the gene *LMNA,* which encodes Lamin A/C. Mutations in *LMNA* are responsible for diseases such as Emery-Dreifuss muscular dystrophy, dilative cardiomyopathy (DCM) and Progeria-Syndrome. Inheritance is typically autosomal-dominant, such that mutation of only one allele is sufficient for a disease phenotype.

Lamin A/C-associated forms of DCM are **characterized** by pronounced fibrosis, arrhythmia and reduced life expectancy (Paldino et al. Curr Cardiol Rep 2018, Pubmed ID (PMID): 30105555). *LMNA* mutations can be found in approximately 6 to 10% of inherited DCM and are thought to be causative (Lu et al. Dis Model Mech 2011 PMID: 21810905, Mazzarotto et al. Circulation 2020, PMID: 31983221).

Currently, the only available therapy for DCM is early implantation of cardioverter/defibrillators or heart transplant. Lamin mutation remains the only approved indicator for prophylactic implantation of cardioverter/defibrillators (Paldino et al. Curr Cardiol Rep 2018, PMID: 30105555).

Due to the great variety in *LMNA* mutations and the complex disease phenotype, therapies specific to only one particular affected signaling cascades are unlikely to yield positive results. Single point mutations can produce a disease phenotype, but the different mutations have diverse molecular consequences. Therefore, correction of single mutations, for example using gene editing methods such as CRISPR/Cas9, is not therapeutically feasible for a diverse group of patients, since it could only be applied to a limited number of patients carrying a specific mutation. A bespoke therapy would have to be designed and tested for each of the 500 known mutations.

Additionally, the *LMNA* gene is not well suited to an exon-skipping or exon-retention strategy, as it is used for Dystrophin or Titin, since the protein is relatively small and contains many functionally and structurally important domains. Overexpression of wild-type and mutant Lamin A in cultured HeLa cells induces accumulation of nuclear aggregates and aberrant Lamin arrangements (Bechert et al. Exp Cell Res 2003, PMID: 12729796).

The object of the invention is therefore to provide a therapeutic approach to treat laminopathies, such as dilated cardiomyopathy, irrespective of the underlying mutation in the *LMNA* gene. The inventors developed a model of dilated cardiomyopathy using human induced pluripotent stem cells (hiPSC) differentiated into engineered heart tissue (EHT) that phenotypically closely resembles patient heart tissue.

Using this model and an isogenic control in which the *LMNA* mutation has been corrected, the inventors were able to develop a novel therapeutic approach of treating cardiac laminopathy. Surprisingly, the inventors were able to treat laminopathy using an adeno-associated virus (AAV) comprising a nucleic acid molecule encoding Lamin A.

**Objectives and Summary of the Invention**

Subjects affected by laminopathy typically carry at least one mutation in one copy of the *LMNA* gene. Since mutations underlying laminopathies may vary from patient to patient, the object of the invention is to provide a therapy that is generally applicable and suitable to a variety of patients with different mutations. The inventors were surprisingly able to treat dilated cardiomyopathy and restore functional and structural properties of affected cardiomyocytes by introducing a nucleic acid molecule expressing Lamin A into affected cells.

Hence, in a first aspect, the invention relates to a nucleic acid molecule for use in the treatment of laminopathy in a subject, comprising a nucleic acid sequence encoding Lamin A.

In one embodiment, the nucleic acid sequence encodes an amino acid that sequence is at least 70% identical to the amino acid sequence according to SEQ ID NO: 1. In one embodiment, the Lamin A is wild-type Lamin A.

In one embodiment, the nucleic acid molecule further comprises a nucleic acid sequence encoding a promoter. In one embodiment, the promoter may be a constitutive promoter, i.e. a promoter with ubiquitous expression activity in eukaryotic cells, or a tissue-specific, e.g. cardiomyocyte-specific promoter. In a preferred embodiment, the tissue-specific promoter is a cardiomyocytes-specific promoter.

Laminopathies are typically caused by mutations in the *LMNA* gene. About 500 different mutations have been described that are linked to laminopathy, over 150 of which are linked to cardiac laminopathies. Hence, in one embodiment, the laminopathy is caused by one or more mutations of the *LMNA* gene.

In one embodiment, the one or more mutations of the *LMNA* gene causes haploinsufficiency for Lamin A or the one or more mutations of the *LMNA* gene causes a dominant-negative Lamin A. For example, the mutation may lead to the expression of a truncated protein and therefore result in haploinsufficiency, or the mutation may be a miss-sense mutation that results in a dominant negative variant of the protein.

Laminopathies can be loosely categorized by their phenotype and affected systems. Generally, *LMNA* mutations affect one or more of striated muscle, adipose tissue, peripheral nerve or multiple systems with features of accelerated aging.

In one embodiment, the laminopathy is a laminopathy that affects striated muscle. Hence, in one embodiment, the laminopathy is selected from cardiomyopathy or muscular dystrophy. In one embodiment, the laminopathy may be Emery-Dreifuss muscular dystrophy, cardiomyopathy, limb-girdle muscular dystrophy or congenital muscular dystrophy. In a preferred embodiment, the laminopathy is cardiomyopathy, more preferably dilated cardiomyopathy.

The nucleic acid molecule of the invention may be comprised in a vector. Hence, in another aspect, the invention relates to a vector comprising the nucleic acid molecule of the invention for use in the treatment of laminopathy in a subject.

In one embodiment, the vector is an expression vector further comprising a nucleic acid sequence encoding a promoter and wherein the promoter is operably linked to the nucleic acid sequence encoding Lamin A, resulting in expression of Lamin A in cells contacted with the vector when the promoter is active.

Mutations in *LMNA* commonly lead to haploinsufficiency and/or dominant-negative activity of the resulting mutant protein. Therefore, the levels of expression of the wild-type allele of Lamin A in affected cells and tissues are usually greatly reduced compared to expression in wild-type cells or organisms. Hence, one objective of the invention is to raise the levels of wild-type Lamin A protein in the affected cells without inducing toxicity or protein aggregation.

Thus, in one aspect of the invention, the expression of Lamin A from the vector increases the overall expression level of Lamin A in cells affected by laminopathy contacted with the vector to at least 50% of the wild-type expression level. Preferably, the expression level of Lamin A increases to between about 60% and about 300% of the wild-type expression level.

Functionally, *LMNA* mutation phenotypes can affect the contractile force of affected cardiomyocytes, increase the risk of beating irregularities (arrhythmia) and increase heart rate in cardiomyocytes affected by laminopathy.

Thus, in one embodiment, the expression of Lamin A from the vector increases the absolute force produced by cardiomyocytes affected by laminopathy contacted with the vector by at least 5% compared to cardiomyocytes not contacted with the vector. In another embodiment, the expression of Lamin A from the vector increases the absolute force produced by cardiomyocytes contacted with the vector by 5% to 200% compared to cardiomyocytes affected by laminopathy not contacted with the vector.

In one embodiment, expression of Lamin A from the vector reduces the degree of beating irregularities by at least 5 % in cardiomyocytes affected by laminopathy contacted with the vector compared to cardiomyocytes affected by laminopathy not contacted with the vector, wherein the beating irregularities are detected by measuring cycle length variability using RR scatter.

In another embodiment, expression of Lamin A from the vector reduces beats per minute by at least 1% in cardiomyocytes affected by laminopathy contacted with the vector compared to cardiomyocytes affected by laminopathy not contacted with the vector.

The vector of the invention may be packaged into a replication-deficient Adeno-associated virus (AAV) or any other virus suitable for delivering gene therapy into cells.

Hence, a further aspect of the invention relates to an AAV for use in the treatment of laminopathy in a subject comprising or the nucleic acid molecule or the vector for use of the invention. In one embodiment, the AAV is serotype AAV6 or AAV9.

In one embodiment, the AAV for use is administered at a titer suitable to induce expression of Lamin A to at least 50% of WT expression. Preferably, the AAV for use is administered at a titer of about 10,000 to about 500,000 virus particles/cell.

Lastly, in one aspect the invention also provides a pharmaceutical composition for use in the treatment of laminopathy in a subject comprising the nucleic acid molecule for use, the vector for use or the AAV for use of the invention and a pharmaceutically acceptable carrier.

## Brief Description of the Drawings

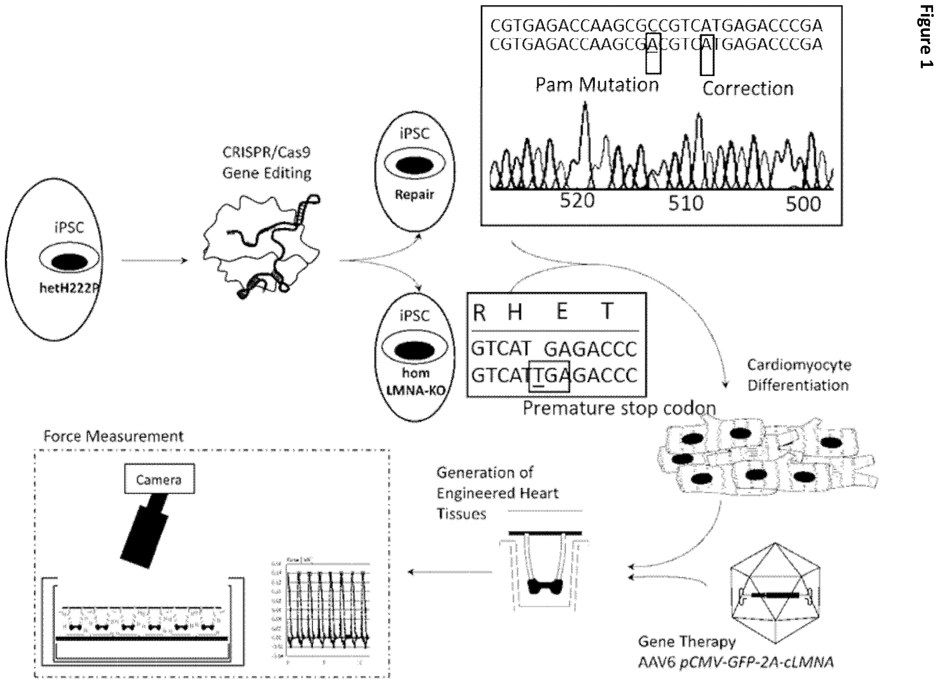

**Figure 1. Experimental strategy.** Induced pluripotent stem cells (iPSC) were reprogrammed from a skin biopsy of a laminopathy patient carrying the heterozygous missense mutation p.H222P in exon 4 (hetH222P). The mutation of the patient cell line hetH222P was corrected via CRISPR/Cas9 gene editing (IsoC, WT sequence according to SEQ ID NO: 15, corrected sequence with Pam mutation according to SEQ ID NO: 16). Simultaneously a clone was generated carrying a homozygous premature stop codon in exon 4 (*LMNA*-KO, partial sequence showing WT sequence according to SEQ ID NO: 17, partial sequence showing premature stop codon according to SEQ ID NO:18). iPSC of the two CRISPR edited cell lines were differentiated into cardiomyocytes (CM) and casted into engineered heart tissues (EHTs). Force development of EHTs were tracked over time. EHTs were treated with an AAV6-mediated gene therapy composing of a CMV promoter, GFP and cDNA encoding for Lamin A separated by a 2A peptide (some elements retrieved from BioRender.com or adapted from Hansen et al. 2010).

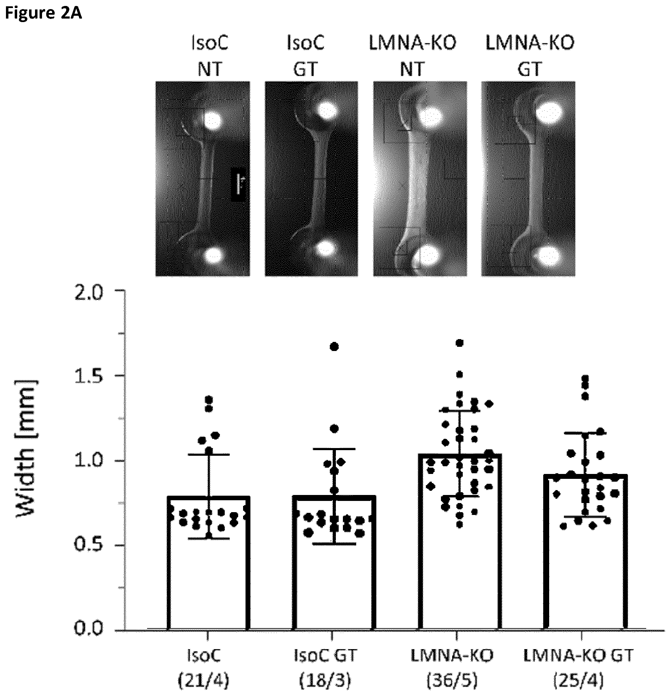

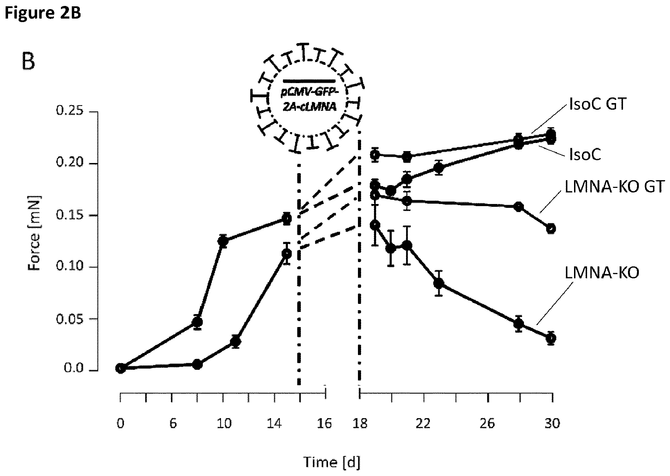

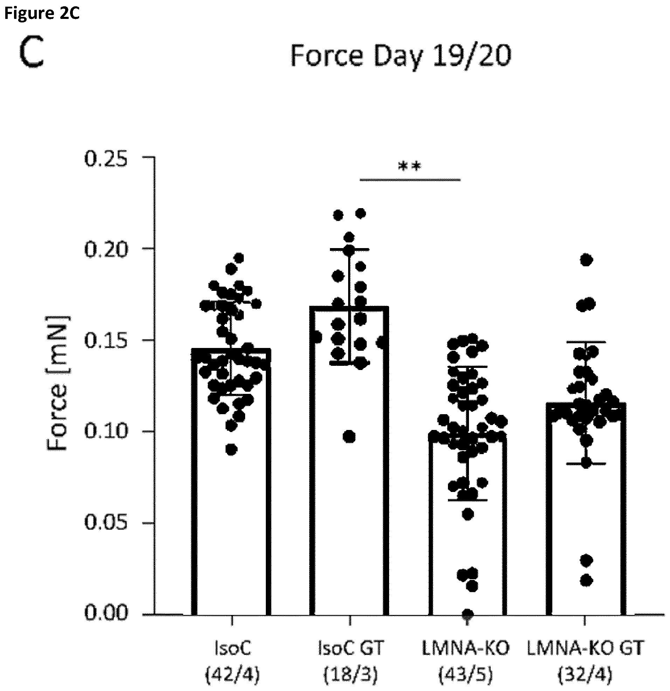

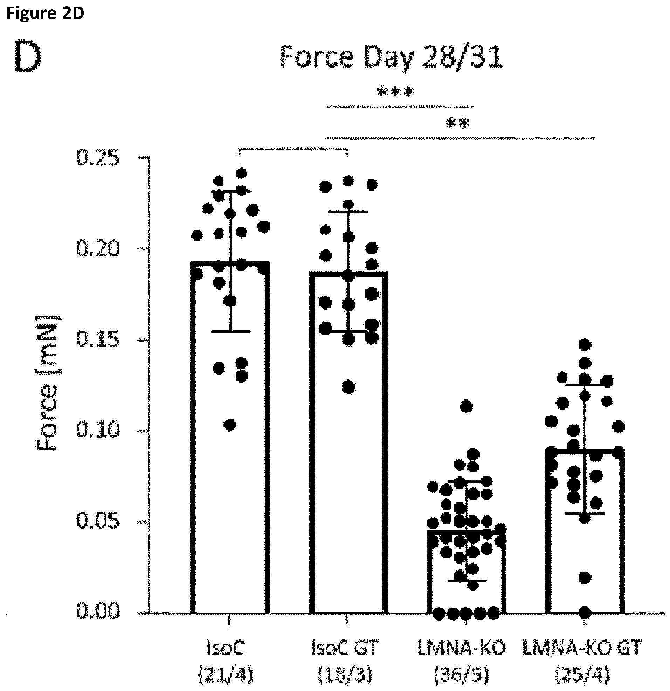

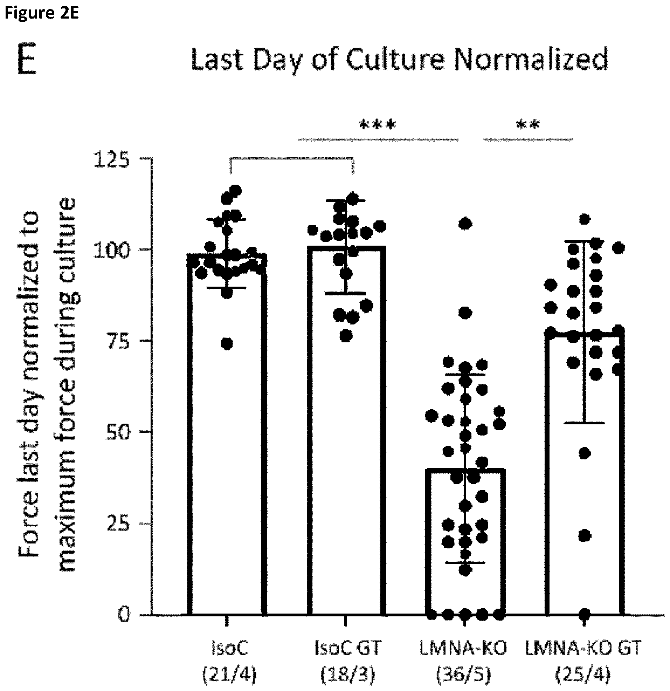

**Figure 2. Remodeling and development of contractile force of EHTs over time.** A Representative images of IsoC and *LMNA*-KO EHTs, either non-transduced (NT) or transduced with AAV6-*LMNA* (GT; last day of culture) EHT remodelling was assessed on last day of culture by manually measuring the width measured video-optically in the centre of the EHT (nested one-way ANOVA non-significant; the investigator was blinded to group assignment). Numbers in brackets indicate number of EHTs (n)/independent batches (N). The batches are indicated by different colours in the dot plots.

B Force development over 30 days of a representative batch of IsoC and *LMNA-KO.* EHTs were transduced with AAV6-*LMNA* (GT) or treated with vehicle on day 15 in culture, n=8 EHTs (*LMNA*-KO and Repair, n=4 (GT), mean±SEM.
C-E Absolute forces measured on day 19/20 (C) and 28/30/31 (D) in culture. Force measured on last day of cultured normalized to maximum mean force during the culture period of each batch (E). N= EHTs/batches, mean $\pm$ SD. Nested one-way ANOVA and Tukeys post-test ** $P_{adj}$ value < 0.01, *** $P_{adj}$ value < 0.005. Numbers in brackets indicate number of EHTs (n)/independent batches (N). The batches are indicated by different colours in the dot plots.

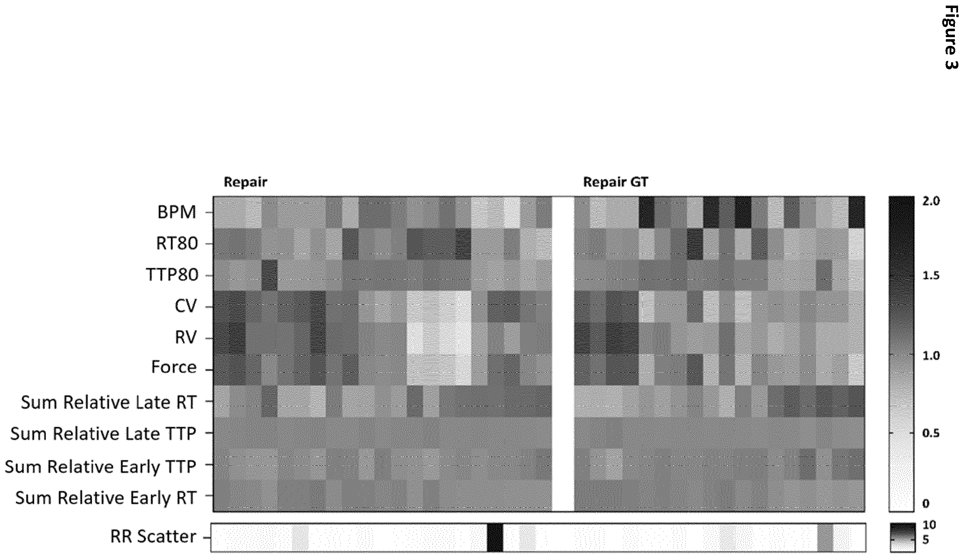

**Figure 3. Effect of Lamin A overexpression on contractile function in control EHTs.** Functional parameters of single EHTs of IsoC (21/4) and IsoC GT (18/3) normalized to the mean of all values. Beats per minute (BPM), relaxation time 80% (RT80), time to peak -80% (TTP80), contraction velocity (CV), relaxation velocity (RV), force, relative late relaxation time, relative late time to peak, relative early time to peak, relative early relaxation time and RR scatter (variability of R-R/beat-to-beat distance).

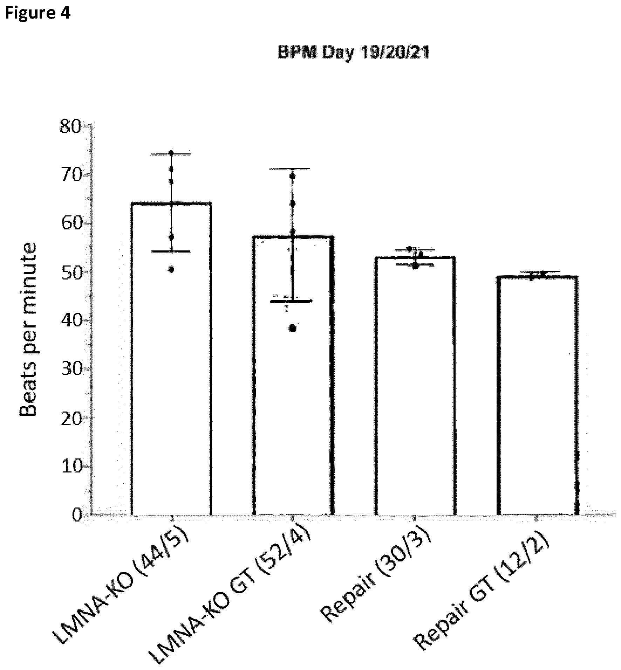

**Figure 4: Gene replacement therapy rescues heart beat frequency and variability phenotypes A** Beats per minute (bpm) of *LMNA*-KO, *LMNA*-KO GT, IsoC and IsoC GT groups measured on days 19/20/21.

**Figure 5**

**A** Principal component analysis (PCA) of IsoC (blue), *LMNA*-KO (red) and *LMNA*-KO GT (green) based on RNA-sequencing analysis. Each symbol represents an EHT.
B Up- and downregulated genes in *LMNA*-KO versus IsoC, *LMNA*-KO versus *LMNA*-KO GT and *LMNA-KO* GT versus IsoC (FDR <0.05).
C Venn diagram of genes differentially expressed in *LMNA-KO* versus IsoC (blue; upper panel) and *LMNA*-KO GT versus IsoC (yellow; upper and lower panel; padj <0.05) and their overlap (shaded; upper panel). Out of the 3486 genes differentially expressed in *LMNA*-KO versus IsoC, a sub-group '*LMNA*-KO GT Enrichment Set' (blue; lower panel) consisting of 2524 genes was corrected upon *LMNA*-KO GT towards IsoC (blue; upper panel; padj <0.05). Out of them, 1043 genes belong to the 3343 differentially expressed in *LMNA-KO* GT versus IsoC (shaded; lower panel).
D Selected enriched gene ontology terms of biological processes of the *LMNA*-KO GT Enrichment Set (red electrophysiology, respiration green, contraction blue; normalised enrichment score (NES); FDR < 0.05).

**Figure 6**

**A** Western blot analysis of EHTs stained with antibodies directed against Lamin A/C, myosin-binding protein C3 (MYBPC3) and green fluorescent protein (GFP).
**B** Quantification of Lamin A and C signal intensity normalized to that of MYBPC3 used as loading control. Data are expressed as mean $\pm$ SD (n=EHTs/batches).
**C** Lamin A/C peptides detected via mass spectrometry (MS) in EHT samples (n=EHTs/batches) normalized to total peptides detected within one sample.
**D** Principal component analysis (PCA) of IsoC (blue), *LMNA*-KO (red circles) and *LMNA*-KO GT (red rectangles in yellow circle) based on MS analysis. Each symbol represents an EHT

(n=EHTs/batches).

E Supervised clustering (*LMNA*-KO vs *LMNA*-KO GT) of proteome profile via MS of the three groups IsoC, *LMNA*-KO, *LMNA*-KO GT (3 EHTs from one batch). Heatmap indicates log2 values of fold change over total detected peptides.

F Selected proteins belonging to cardiac contraction or Lamin interactome, up- and downregulated (log2fold change from mean of all groups) in single EHTs of IsoC *LMNA*-KO and *LMNA-KO* GT. Direct Lamin A/C interactions partners are presented in bold (n=EHTs/batches; *LMNA*-KO versus IsoC p value < 0.05, t-test).

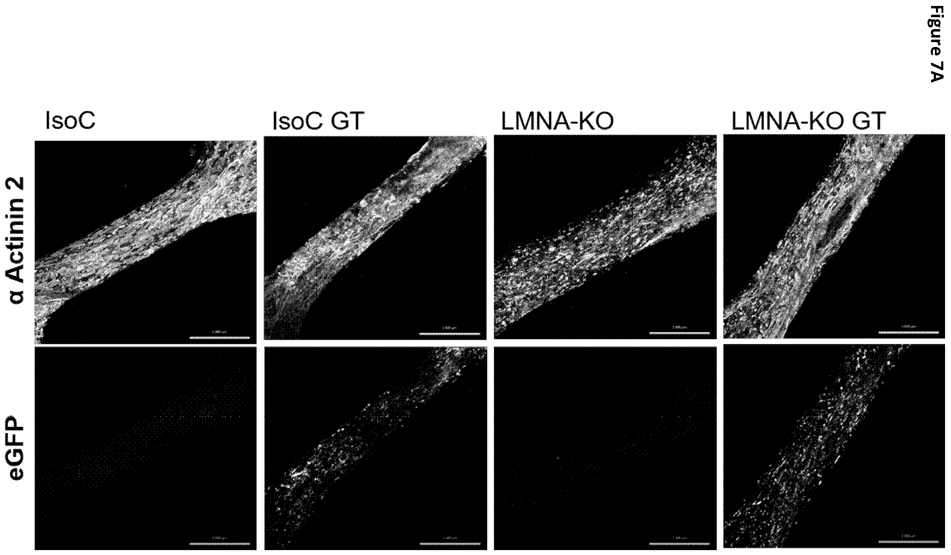

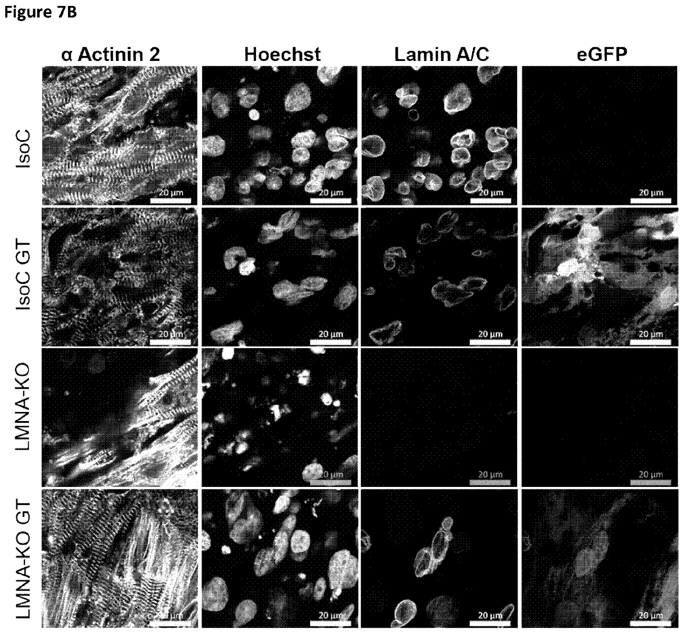

**Figure 7. Structural disintegration in *LMNA*-KO rescued by *LMNA* overexpression.**

**A** Immunostaining of human cardiac $\alpha$-actinin-2 and green fluorescent protein (GFP) of IsoC, IsoC GT, *LMNA*-KO and *LMNA*-KO GT EHTs after at least 30 days in culture (scale 1,000 $\mu$m).

**B** Representative images of IsoC, IsoC GT, *LMNA*-KO and *LMNA*-KO GT EHTs stained for human cardiac $\alpha$-actinin-2, Lamin A/C and nuclei (Hoechst; scale 20 $\mu$m). The images are representative of 3 independent EHTs.

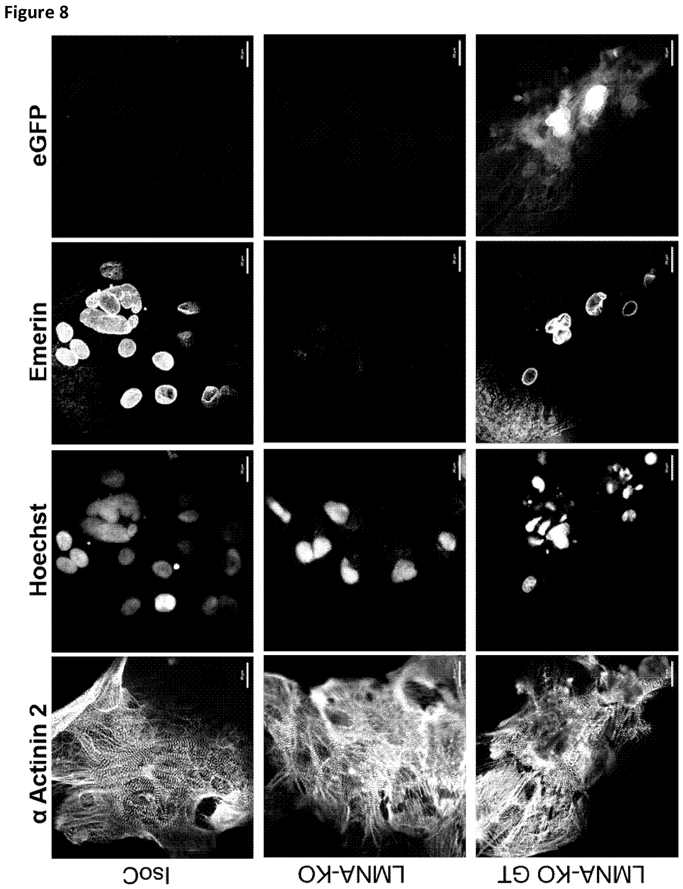

**Figure 8.** Restoration by *LMNA* overexpression of emerin in the nuclear envelope. Immunofluorescence analysis of 2D cultured iPSC-CMs stained for human cardiac $\alpha$-actinin-2, nuclei (Hoechst) and emerin (scale 20 $\mu$m).

## DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable".

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of $\pm$10%, and preferably of $\pm$5%.

Technical terms are used by their common sense. If a specific meaning is conveyed to certain terms, definitions of terms will be given in the following in the context of which the terms are used.

### Nucleic acid molecule encoding Lamin A

The terms "nucleic acid" or "nucleic acid molecule" or "nucleic acid sequence" or "nucleotide sequence" are used interchangeably herein to refer to a biomolecule composed of nucleotides. The nucleic acid molecule can be comprised within an eukaryotic or prokaryotic organism, a eukaryotic or prokaryotic cell, a cell nucleus or a cell organelle, as part of a genome or as an individual molecule; or it can be comprised within a plasmid, a vector, an artificial chromosome; a nucleic acid can also exist outside of a cell, in vesicles, viruses or freely circulating, it can be isolated in a suitable composition, in a fixed or frozen tissue or cell culture, or dried. The nucleic acid can be synthesized or naturally occurring, i.e. isolated from nature.

The terms "encoded protein" or "encoded amino acid" refers a protein that consists of a chain of amino acids, which results from a sequence that is encoded by a nucleic acid molecule comprising three-nucleotide codons.

As used herein, the term "laminopathy" or "laminopathies" refers to a group of genetic disorders caused by mutations in genes encoding proteins of the nuclear Lamina. The nuclear Lamina is an intermediate filament network that provides scaffolding for the cell nucleus. Laminopathy belongs to a group of diseases termed "nuclear envelopathies" and disease-causing mutations may occur in nuclear envelope proteins including Emerin (encoded in humans by the gene *EMD*), MAN1 (encoded in humans by the gene *LEMD3*), Lamin A/C (encoded in humans by the gene *LMNA*), Lamin B 1 and 2 (encoded in humans by the genes *LMNB1* and *LMNB2*) and Lamin B receptor (encoded in humans by the gene *LBR*). Thus, nuclear envelopathies involving Lamins are collectively called laminopathies.

Laminopathies can be loosely categorized by their phenotype and affected systems. Generally, *LMNA* mutations affect one or more of striated muscle, adipose tissue, peripheral nerve or multiple systems with features of accelerated aging.

"Striated muscle" can be either cardiac muscle or skeletal muscle and the term refers to a muscle tissue that, as opposed to smooth muscle, features repeating functional units called sarcomeres. Hence, laminopathy phenotypes commonly affect both heart muscle and skeletal muscle.

The term "muscular dystrophy" refers to a group of muscle disorders that are caused by genetic mutations, usually in genes encoding muscle cell-specific proteins. Muscular dystrophies cause progressive weakening and breakdown of muscle tissue which leads to a range of symptoms, including inability to walk, limited range of movement, respiratory difficulty, muscle spasms, deformations and cardiomyopathy. Muscular dystrophies associated with mutations in *LMNA* include autosomal dominant and autosomal recessive Emery-Dreifuss muscular dystrophy, congenital muscular dystrophy, dilated Cardiomyopathy (or "Cardiomyopathy dilated 1A") and limb-girdle muscular dystrophy type 1B.

The term "cardiomyopathy" refers to a disease or dysfunction of heart muscle.

Laminopathies caused by *LMNA* mutation with adipose tissue involvement, called partial lipodystrophy syndromes, include Dunnigan-type familial partial lipodystrophy, lipoatrophy with diabetes, hepatic steatosis, hypertrophic cardiomyopathy and leukomelanodermic papules, insulin resistance without lipoatrophy and mandibuloacral dysplasia.

*LMNA* mutations involving peripheral nerve tissue or "peripheral neuropathy" include Charcot-Marie-Tooth disorder type 2B1. Premature aging syndromes caused by *LMNA* mutations include Hutchinson-Gilford progeria syndrome, Restrictive dermopathy, variant progeroid disorders, mandibuloacral dysplasia (this disease has features of both lipodystrophy and progeria) and atypical Werner syndrome.

In one embodiment, the laminopathy is a laminopathy that affects striated muscle. Hence, in one embodiment, the laminopathy is selected from Emery-Dreifuss muscular dystrophy, cardiomyopathy, limb-girdle muscular dystrophy 1B and congenital muscular dystrophy. In a preferred embodiment, the laminopathy is cardiomyopathy, more preferably dilated cardiomyopathy 1A. Preferably, the laminopathy is not a peripheral neuropathy.

"Dilated cardiomyopathy" or "DCM" as used herein refers to a condition in which the heart becomes enlarged and fails to pump blood effectively, eventually resulting in heart failure. About 20-25% of cases of DCM are familial, i.e. they result from inherited mutations. Affected genes include *LMNA* encoding Lamin A/C, *SCN5A* encoding Na$_V$1.5, *TNNT2* encoding cardiac muscle Troponin, *TTN* encoding Titin, *DES* encoding Desmin, *ACTC* encoding cardiac muscle α-Actin, *PLN* encoding Phospholamban, and many others. DCM associated with mutations in *LMNA* is referred to as "dilated cardiomyopathy 1A" or "CMD1A".

The "Lamin A" protein (UniProt accession number: P02545) referred to herein is one of two splice variants of the protein encoded by the *LMNA* gene, the other being Lamin C. Lamin A and C are type 5 intermediate filaments and localize to the nuclear Lamina, a stabilizing layer underneath the inner surface of the nuclear envelope. Nuclear Lamins have a variety of functions including structural support of the nuclear envelop, providing anchorage sites for chromatin, nuclear envelope assembly, DNA synthesis, transcription and apoptosis. Each Lamin subtype forms separate Lamin polymers and filaments in the nuclear Lamina. Additionally, Lamins can also be found in the nuclear interior in soluble, unpolymerized form.

*LMNA* encodes several splice isoforms, including Lamin A (664 amino acids) and Lamin C (576 amino acids). The two splice variants Lamin A and C share the first 566 amino acids but differ at the carboxyl-terminus, where Lamin A contains an additional two exons that are lacking in Lamin C. Nuclear Lamins are targeted to the inner nuclear membrane by nuclear localization motifs. Lamin A is first expressed as a precursor protein (PreLamin A), and then undergoes posttranslational modification. PreLamin A contains a CaaX motif which triggers farnesylation and methylation, and also undergoes an additional endoproteolytic processing step at which the last 15 amino acids of the protein are clipped off, resulting in mature Lamin A. The "CaaX motif" is a common terminal acid motif in cellular proteins and consists of a cysteine residue followed by two aliphatic amino acid residues and another variable amino acid residue.

The first aspect of the invention is providing a nucleic acid molecule for use in the treatment of laminopathy in a subject, comprising a nucleic acid sequence encoding Lamin A.

*LMNA* produces a variety of transcripts, encoding at least six known isoforms. The canonical sequence encodes a protein of 664 amino acids which is represented by the amino acid sequence according to SEQ ID NO: 1. The mature Lamin A protein after post-translational processing consists of amino acids 1 to 646 of SEQ ID NO: 1 and is represented by the amino acid sequence according to SEQ ID NO: 2. A nucleic acid sequence encoding SEQ ID NO: 1 is represented by the nucleic acid sequence according to SEQ ID NO: 3.

In one embodiment, the nucleic acid molecule encodes an amino acid that comprises an amino acid sequence that is at least 70% identical to the amino acid according to SEQ ID NO: 1. In one embodiment, the Lamin A is wild-type Lamin A. In one embodiment, the nucleic acid molecule encodes an amino acid that comprises an amino acid sequence that is at least 75%, at least 80%, at least 85%, at least 90%, at least 95%, at least 98%, at least 99% or 100% identical to the amino acid according to SEQ ID NO: 1. In one embodiment, the nucleic acid molecule encodes an amino acid that comprises an amino acid sequence according to any one of SEQ ID NO: 1.

In another embodiment, the nucleic acid molecule encodes an amino acid that comprises an amino acid sequence that is at least 70% identical to the amino acid sequence according to SEQ ID NO: 2. In one embodiment, the nucleic acid molecule encodes an amino acid that comprises an amino acid sequence that is at least 75%, at least 80%, at least 85%, at least 90%, at least 95%, at least 98%, at least 99% or 100% identical to the amino acid according to SEQ ID NO: 2. In one embodiment, the nucleic acid molecule encodes an amino acid that comprises an amino acid sequence according to any one of SEQ ID NO: 2.

In another embodiment, the nucleic acid of molecule comprises a nucleic acid molecule that is at least 70% identical to the nucleic acid sequence according to SEQ ID NO: 3. In another embodiment, the nucleic acid of molecule comprises a nucleic acid molecule that is at least 75%, at least 80%, at least 85%, at least 90%, at least 95%, at least 98%, at least 99% or 100% identical to the nucleic acid sequence according to SEQ ID NO: 3. In another embodiment, the nucleic acid of molecule comprises a nucleic acid sequence according to SEQ ID NO: 3.

The nucleic acid of the invention may encode any wild-type isoform of Lamin A. Thus, in some embodiments, the nucleic acid molecule encodes an amino acid that comprises an amino acid sequence that is at least 70% identical to the amino acid sequence according to any one of SEQ ID NOs: 4 - 8. In one embodiment, the nucleic acid molecule encodes an amino acid that comprises an amino acid sequence that is at least 75%, at least 80%, at least 85%, at least 90%, at least 95%, at least 98%, at least 99% or 100% identical to the amino acid sequence according to any one of SEQ ID NOs: 4 - 8. In one embodiment, the nucleic acid molecule encodes an amino acid that comprises an amino acid sequence according to any one of SEQ ID NOs: 4 - 8.

In one embodiment, the nucleic acid molecule encodes an amino acid that comprises an amino acid sequence that is at least 70% identical to the amino acid sequence according to any one of SEQ ID NOs: 1, 2, 4, 5, 6, 7 or 8.

The terms "sequence Identity", "% sequence identity", "% identity", "% identical" or "sequence alignment" are used interchangeably herein and refer to the comparison of a first nucleic acid sequence to a second nucleic acid sequence, or a comparison of a first amino acid sequence to a second amino acid sequence and is calculated as a percentage based on the comparison. The result of this calculation can be described as "percent identical" or "percent ID." A sequence identity may be determined by a program, which produces an alignment, and calculates identity counting both mismatches at a single position and gaps at a single position as non-identical positions in final sequence identity calculation. The sequence identity is determined over the entire length of the first and second nucleic acid sequence.

The terms "polypeptide", "protein" or "peptide" are used interchangeably herein and refer to amino acid sequences of a variety of lengths. The term polypeptide may also refer to the primary, secondary, tertiary, quarternary or quinary structure or a protein. Polypeptides can be in uncharged forms or as salts, either unmodified or modified by glycosylation, side chain oxidation, phosphorylation, citrullination or transglutamination. In certain embodiments, the polypeptide is a full-length native protein. In other embodiments, the polypeptide is a smaller fragment of the full-length protein. In still other embodiments, the amino acid sequence is modified by additional substituents attached to the amino acid side chains, such as N- or C-terminal added protein tags, glycosyl units, lipids, or inorganic ions such as phosphates, as well as modifications relating to chemical conversion of the chains such as oxidation of sulfhydryl groups. Thus, the term "polypeptide" is intended to include the amino acid sequence of the full-length native protein, or a fragment thereof, subject to those modifications that do not significantly change its specific properties. In particular, the term "polypeptide" encompasses protein isoforms, i.e., variants that are encoded by the same gene, but that differ in their amino acid sequence or in other properties.

The "UniProt numbers" or "UniProt" or "UniProt Accession numbers" provided herein refer to the unique identifiers given to individual genes and proteins by the UniProt Consortium, which are available from their database at www.uniprot.org and commonly used as references in the field. UniProtKB (UniProt Knowledgebase) is a freely accessible database of protein sequence and functional information. The UniProt database includes manually annotated and reviewed entries (provided by the Swiss-Prot database) and automatically annotated and not manually reviewed entries (provided by TrEMBL database), many of which are derived from genome sequencing projects. TrEMBL includes translated coding sequences from the EMBL-Bank/GenBank/DDBJ nucleotide sequence database, and others.

In one embodiment, the nucleic acid molecule further comprises a nucleic acid sequence encoding a promoter. In one embodiment, the promoter may be a promoter with constitutive activity in eukaryotic cells, or a promoter that is tissue specific.

The term "promoter" refers to a DNA sequence capable of starting and controlling the expression of a coding sequence or functional RNA. Typically, a coding sequence is located 3' to a promoter sequence. Promoters may be derived in their entirety from a native gene, or be composed of different elements derived from different promoters found in nature, or even comprise synthetic DNA segments. Typically, since the exact boundaries of regulatory sequences have not been completely defined, DNA fragments of different lengths may have identical promoter activity. It is understood by a person skilled in the art that different promoters may direct the expression of a gene at different stages of development, or in response to different environmental or physiological conditions. Promoters that cause a gene to be expressed in most cell types at most times are commonly referred to as constitutive promoters. On the other hand, promoters that cause a gene to be expressed in specific contexts only, e.g. based on the presence of specific factors, growth stages, temperatures, pH or the presence of specific metabolites etc. are understood as regulatable promoters.

The term "3' non-coding sequences" or "5' non-coding sequences " or "3' UTR" or "5' UTR" refers to DNA sequences located downstream of a coding sequence. The term "RNA transcript" refers to the product resulting from RNA polymerase catalyzed transcription of a DNA sequence. The term "mRNA" refers to messenger RNA, i.e. RNA that is without introns and that can be translated into proteins by the cell. The term "cDNA" refers to complementary DNA, which means a DNA that is generated from an RNA by reverse transcription.

The term "operably linked" refers to the association of nucleic acid sequences on a single nucleic acid fragment so that the function of one is affected by the other. In the context of a promoter the term means that the coding sequence is under the transcriptional control of the promoter which regulates the expression of the coding sequence.

A "constitutive promoter" or "ubiquitous promoter" as used herein is a promoter active *in vivo* in all circumstances. The expression from a constitutive promoter is non-selective.

In contrast, a "selective" or "tissue-specific" promoter is a promoter that is specifically activated in one or more cell types or tissues and is not active in other cell types. For example, promoters of cardiac-specific genes are active in cardiac tissues, specifically in cardiomyocytes. Hence, in one embodiment, the promoter is a cardiac-specific promoter. In one embodiment, the cardiac-specific promoter is selected from the group consisting of myosin light chain 2 (MLC-2v) promoter, alpha myosin heavy chain ($\alpha$MHC) promoter, beta myosin heavy chain ($\beta$MHC) promoter cardiac Troponin T (cTnT) promoter, myocyte-specific enhancer factor 2C (MEF2C) promoter and Nk2 homeobox 5 (Nkx2.5) promoter. Preferably, the promoter is selected from myosin light chain 2 (MLC-2v) promoter, alpha myosin heavy chain ($\alpha$MHC) promoter, beta myosin heavy chain ($\beta$MHC) promoter cardiac Troponin T (cTnT) promoter, and myocyte-specific enhancer factor 2C (MEF2C) promoter. Most preferably, the promoter is a cardiac Troponin T promoter. In another embodiment, the promoter is a Lamin A/C promoter.

In another embodiment, the promoter may be a constitutive promoter, a tissue-specific promoter or an inducible promoter.

"Inducible promoters" are promoters that can be switched on and off. They can be chemically inducible, such as the tetracycline inducible Tet operator, lac operator, Gal4-UAS system or inducible Cre-lox system, temperature inducible, such as Hsp70 or Hsp90, or light inducible. Cardiac specific promoters can be combined with inducible enhancers or promoter elements for time and space specific inducible activity. Thus, in one embodiment, the promoter may be a cardiac specific inducible promoter.

The promoter may be an ubiquitous promoter or any suitable promotor for expression in the chosen system, for example CMV (cytomegalovirus), T7, lac operator, EF1alpha (elongation factor 1 alpha), chicken beta-Actin, CAG, Tet operator, EpIE2, SV40 (simian virus 40), MT (metallothioneine), UbC (ubiquitin c), RSV (rous sarcoma virus), AUG1, CMV/EF1alpha, FLD1 (formaldehyde dehydrogenase 1), TK (thymidine kinase), Gal4 UAS/E1b (galactose), trc, tac, AOX1 (alcohol oxidase 1), olyhedrin or copia promoter.

In one embodiment, the promoter is a constitutive promoter selected from the group consisting of CMV, CAG, Chicken beta-Actin, EF1alpha, CMV/EF1alpha, and SV40.

In one embodiment, the nucleic acid molecule further comprises a nucleic acid sequence encoding an epitope tag, preferably selected from His-tag, GS-tag, FLAG-tag, c-Myc tag or HA-tag or a fluorescent tag, such as a fluorescent protein or FRET label, or a radiolabel or an antibody tag.

In another embodiment, the nucleic acid molecule further comprises a nucleic acid sequence encoding a linker. The linker may be a fusion linker, a 2A peptide or an internal ribosome entry site (IRES). In one embodiment, the linker is selected from Gs linker, GGGGS linker, T2A, P2A, F2A, E2A and IRES.

Laminopathies are typically caused by mutations in the *LMNA* gene. About 500 different mutations have been described that are linked to laminopathy, distributed along the whole gene without any obvious hotspots, and over 150 of mutations are linked to cardiac laminopathies. Hence, in one embodiment, the laminopathy is caused by one or more mutations of the *LMNA* gene.

The term "mutation" as used herein refers to alterations in the nucleotide sequence of the genome of an organism. Specifically, the term *"LMNA* mutation" refers to alterations in the genomic DNA sequence of *LMNA*. Genes can for example be disrupted by large-scale genomic events, such as chromosomal deletions, rearrangements, inversions or crossovers. These large-scale events can lead to loss of heterozygosity if they only affect one allele, i.e. one copy of the gene.

Small-scale mutations affect a gene in one or only a few nucleotides. Mutations that affect a gene in only one nucleotide are called point mutations. Small-scale or point mutations include insertions, deletions, and substitution mutations.

The following terms describe mutations on the nucleotide sequence level:
"Insertions" refers to the addition of one or more nucleotides into the DNA, while "deletions" refers to the removal of one or more nucleotides from the DNA. Collectively, this type of mutation is also called "indel". Indels can be caused by transposable elements, errors during replication or DNA damage. Indels typically cause frame-shifts in the reading frame of the gene and therefore lead to missense or nonsense mutations. Indels can also affect the splicing of mRNA if they occur in the splice sites, usually at the exon-intron boundaries, and involve the splice donator or splice acceptor nucleotides.

"Frame-shift" mutations occur if the indel involves a number of nucleotides that is not a multiple of three. Because of the three-nucleotide code, inserting or deleting a number of nucleotides that is not a multiple of three changes the amino acid sequence of the encoded protein downstream of the mutation. If such a severe change of sequence occurs early on in the protein sequence, this usually results in a nonfunctional protein. The nonfunctional protein may then be degraded by the cell.

A "missense" mutation causes the substitution of a different amino acid due to a change in the nucleotide codon sequence. A "nonsense" mutation refers to a mutation that results in a premature stop codon, which in turn often leads to a truncated version of the protein being expressed. Missense and nonsense mutations are also called "non-synonymous", which means that they replace a codon with a codon that encodes a different amino acid, thereby changing the sequence.

A "synonymous" or "silent" mutation is a nucleotide mutation that does not change the encoded amino acid, due to the degenerate amino acid code. This typically occurs if only the third nucleotide is mutated, the so-called "wobble base". In one embodiment, the nucleic acid sequence encoding wild-type Lamin A may comprise one or more synonymous mutations.

On the protein sequence level, mutations are usually described based on their functional effect. "Loss-of-function" mutations result in the gene product or protein having less or no function. A complete loss of function may be called a "null allele". Loss of function mutations which result in the reduced expression or no expression of one of the two alleles of a gene cause haploinsufficiency if the reduced dosage of the protein is not enough for a normal or wild-type phenotype.

The term "haploinsufficiency" as used herein refers to a condition in which at least one of two alleles of a gene is mutated and wherein the combined gene product expression level of the wild-type and mutated alleles is not sufficient to produce a wild-type phenotype.

"Gain-of-function" mutations change the gene product or protein such that either its effect is stronger (enhanced activation) or is replaced by a different or abnormal function. "Dominant-negative" mutations are mutations in which the altered gene product or protein acts antagonistically to the wild-type allele and adversely affects the wild-type gene product within the same cell. This occurs for example when a mutation in a protein removes a functional domain but retains DNA or protein-protein interaction domains. In this case, the protein can still bind its interaction partner but does not function, thereby effectively blocking the pathway from functioning normally.

Gain- and loss-of-function mutations can occur within the coding regions of a gene or within the regulatory regions, such as enhancer binding sites or promoter regions. Mutations in regulatory regions may lead to increased or decreased gene expression or changes in the spatio-temporal pattern of gene expression.

Mutations in *LMNA* typically include truncating mutations, which are generally associated with a more severe phenotype, and missense mutations (Crasto et al. Front Physiol 2018, PMID: 32719615). Mutant variants or the partial absence of Lamin A may lead to a disruption of the structure and function of the nuclear Lamina and disrupt both the import and export of molecules through the nuclear pores as well as transcription. Disruption of transcription may be due to the nuclear membrane's function in assembly of densely packaged chromatin complexes in Lamin-associated domains (LADs), which result in a stable suppression of transcription in the affected chromosome segments. The nuclear membrane, and Lamin in particular, also play an important role in mechanotransduction, i.e. the transduction of mechanical stimuli and their effect on transcription (Crasto et al. Front Physiol 2018, PMID: 32719615). This could explain the fact that laminopathies predominantly affect tissues which experience mechanical stress, such as heart or skeletal muscle.

In one embodiment, the one or more mutations of the *LMNA* gene cause haploinsufficiency for Lamin A or the one or more mutations of the *LMNA* gene cause a dominant-negative Lamin A.

For example, the mutation may lead to the expression of a truncated protein and therefore result in haploinsufficiency, or the mutation may be a missense mutation that results in a dominant negative variant of the protein.

Methods of detecting mutations are known in the art and include, for example, sequencing of exons and intron-exon junctions of the *LMNA* gene or copy-number-variation studies.

## Vector for use in gene replacement therapy

The nucleic acid molecule of the invention can be packaged into one or more vectors, e.g., plasmids or viral vectors. In some embodiments, the vectors, e.g., plasmids or viral vectors, are delivered to the tissue of interest by, e.g., intramuscular injection, intravenous administration, transdermal administration, intranasal administration, oral administration, or mucosal administration. Such delivery may be either via a single dose or multiple doses. One skilled in the art understands that the actual dosage to be delivered herein may vary greatly depending upon a variety of factors, such as the vector choices, the target cells, organisms, tissues, the general conditions of the subject to be treated, the degrees of transformation/modification sought, the administration routes, the administration modes, the types of transformation/modification sought, etc.

As used herein, the term "vector" refers to a nucleic acid molecule capable of transporting another nucleic acid to which it has been linked and may be used herein interchangeably with the term "recombinant nucleic acid molecule". One type of vector is a "plasmid", which refers to a circular double stranded DNA loop into which additional DNA segments can be ligated. In the present specification, "plasmid" and "vector" can be used interchangeably as the plasmid is the most commonly used form of vector. The term "expression vector" means a vector capable of directing expression of a particular nucleotide sequence in an appropriate host cell. An expression vector comprises a regulatory nucleic acid element operably linked to a nucleic acid of interest, which is optionally operably linked to a termination signal and/or other regulatory element.

Examples of other expression vectors and host cells are the pET vectors (NOVAGEN), pGEX vectors (Amersham Pharmacia), and pMAL vectors (New England labs. Inc.) for protein expression in *E. coli* host cells such as BL21, BL21(DE3) and AD494(DE3)pLysS, Rosetta (DE3), and Origami(DE3) (NOVAGEN); the strong CMV promoter-based pcDNA3.1 (INVITROGEN) and pCIneo vectors (Promega) for expression in mammalian cell lines such as CHO, COS, HEK-293, Jurkat, and MCF-7; replication incompetent adenoviral vector vectors pADENO X, pAd5F35, pLP- ADENO-X-CMV (CLONTECH), pAd/CMV/V5-DEST, pAd-DEST vector (INVITROGEN) for adenovirus- mediated gene transfer and expression in mammalian cells; pLNCX2, pLXSN, and pLAPSN retrovirus vectors for use with the RETRO-X™ system from Clontech for retroviral-mediated gene transfer and expression in mammalian cells; pLenti4/V5-DEST™, pLenti6/V5-DEST™, and pLenti6.2/V5-GW/lacZ (INVITROGEN) for lentivirus-mediated gene transfer and expression in mammalian cells; adenovirus-associated virus expression vectors such as pAAV- MCS, pAAV-IRES-hrGFP, and pAAV-RC vector (STRATAGENE) for adeno- associated virus- mediated gene transfer and expression in mammalian cells.

The term "coding sequence" refers to a DNA sequence which codes for a specific amino acid sequence. The term "regulatory sequence" refers to a nucleotide sequence located upstream (5' non-coding sequences), within, or downstream (3' non-coding sequences) of a coding sequence, and which influences the transcription, RNA processing or stability, or translation of the associated coding sequence. Regulatory sequences may include promoters, enhancers, translation leader sequences, introns, polyadenylation recognition sequences, RNA processing sites, effector binding sites and stem-loop structures.

The term "expression" or "gene expression" as used herein refers to the process of synthesis of a gene product, preferably a functional RNA or protein. Gene expression generally comprises DNA transcription, optionally RNA processing and in the case of protein-expressing genes, RNA translation.

The nucleic acid molecule of the invention may be comprised in a vector. Hence, in another aspect, the invention relates to a vector comprising the nucleic acid molecule of the invention for use in the treatment of laminopathy in a subject.

In one embodiment, the vector comprises a nucleic acid molecule encoding an amino acid sequence that is at least 70% identical to the amino acid sequence according to SEQ ID NO: 1. In one embodiment, the vector comprises a nucleic acid molecule encoding an amino acid sequence that is at least 75%, at least 80%, at least 85%, at least 90%, at least 95%, at least 98%, at least 99% or 100% identical to the amino acid sequence according to SEQ ID NO: 1.

In another embodiment, the vector comprises a nucleic acid molecule encoding an amino acid sequence that is at least 70% identical to the amino acid sequence sequence according to SEQ ID NO: 2. In one embodiment, the vector comprises a nucleic acid molecule encoding an amino acid sequence that is at least 75%, at least 80%, at least 85%, at least 90%, at least 95%, at least 98%, at least 99% or 100% identical to the amino acid sequence according to SEQ ID NO: 2.

In another embodiment, the vector comprises a nucleic acid molecule comprising a nucleic acid sequence that is at least 70% identical to the nucleic acid sequence according to SEQ ID NO: 3. In another embodiment, the vector comprises a nucleic acid molecule comprising a nucleic acid sequence that is at least 75%, at least 80%, at least 85%, at least 90%, at least 95%, at least 98%, at least 99% or 100% identical to the nucleic acid sequence according to SEQ ID NO: 3. In another embodiment, the vector comprises a nucleic acid molecule comprising a nucleic acid sequence according to SEQ ID NO: 3.

The vector may comprise a nucleic acid molecule encoding any wild-type isoform of Lamin A. Thus, in some embodiments, the vector comprises a nucleic acid molecule encoding an amino acid sequence that is at least 70% identical to the amino acid sequence according to any one of SEQ ID NOs: 4 - 8. In one embodiment, the vector comprises a nucleic acid molecule encoding an amino acid sequence that is at least 75%, at least 80%, at least 85%, at least 90%, at least 95%, at least 98%, at least 99% or 100% identical to the amino acid sequence according to any one of SEQ ID NOs: 4 - 8. In one embodiment, the vector comprises a nucleic acid molecule encoding an amino acid sequence according to any one of SEQ ID NOs: 4-8.

In one embodiment, the vector comprises a nucleic acid molecule encoding an amino acid sequence according to any one of SEQ ID NOs: 1, 2, 4, 5, 6, 7 or 8.

In one embodiment, the vector is an expression vector comprising a nucleic acid molecule comprising a nucleic acid sequence encoding Lamin A and further comprising a nucleic acid sequence encoding a promoter. In a preferred embodiment, the nucleic acid sequence encoding the promoter is operably linked to the nucleic acid sequence encoding Lamin A. Typically, contacting the cells with the vector results in expression of Lamin A in said cells. Preferably, expression of Lamin A in cells contacted with the vector occurs when the promoter is active.

A promoter is considered "active" when gene expression, i.e. transcription, from the promoter occurs. This may occur when the promoter is bound by RNA polymerase and/or activators and/or enhancers. Promoter activity can be measured by methods known in the art, such as determining the gene product, mRNA or protein, or expressing a reporter gene product.

As used herein, the term "level" refers to the quantity of a specific molecule in a biological sample. The term "expression" or "gene expression" as used herein refers to the process of synthesis of a gene product, preferably a functional RNA or protein. Gene expression generally comprises DNA transcription, optionally RNA processing and in the case of protein-expressing genes, RNA translation.

"Expression", "expression level", "gene expression" or "gene expression level" can be measured by determining mRNA (transcription) or protein (translation) levels. Methods to measure mRNA or protein levels are known in the art and may involve relative or absolute quantification. For example, absolute quantification of mRNA can be achieved by RNA sequencing, while relative quantification of mRNA can be achieved by quantitative PCR; absolute quantification of protein can be achieved by mass spectrometry or enzyme-linked immunosorbent assay (ELISA), while relative quantification and/or presence or absence of protein can be achieved by immunoblotting (Western Blot) or immunohistochemistry. Gene expression can also be measured using reporter assays such as luciferase assay. The level may be determined numerically, or it may be determined by measuring a secondary signal, such as fluorescence intensity. The level may also be expressed binary by presence or absence of the measured molecule, i.e. the level may be positive or negative.

Since mutations in *LMNA* may lead to haploinsufficiency or dominant-negative activity of the resulting mutant protein, the expression levels of functional Lamin A expression in affected cells are usually reduced compared to wild-type expression. This may be due to expression of a nonfunctional allele of Lamin A, or due to expression of a dominant-negative allele of Lamin A. Since Lamins polymerize into filaments, dominant-negative Lamins can also affect the functionality of wild-type Lamins expressed in the same cell, thereby further reducing the concentration or level or available functional Lamins. This may occur for example through aggregation of Lamin. Hence, one objective of the invention is to raise the levels of wild-type Lamin A protein in the affected cells.

Thus, in one aspect of the invention, the expression of Lamin A from the vector increases the overall expression level of Lamin A in cells affected by laminopathy contacted with the vector to at least 50% of the wild-type expression level.

In one embodiment, the expression of Lamin A from the vector increases the overall expression level of Lamin A in cells affected by laminopathy contacted with the vector to at least 60%, at least 70%, at least 80%, at least 90%, at least 100%, at least 110%, at least 120%, at least 130%, at least 140%, at least 150%, at least 160%, at least 170%, at least 180%, at least 190%, at least 200%, at least 210%, at least 220%, at least 230%, at least 240%, at least 250%, at least 260%, at least 270%, at least 280%, at least 290%, or at least 300%, of the wild-type expression level.

In one embodiment, the expression of Lamin A from the vector increases the overall expression level of Lamin A in cells affected by laminopathy contacted with the vector to no more than 300% of wild-type expression level. Hence, in one embodiment, the expression level of Lamin A increases to between about 50% and about 300% of the wild-type expression level.

In another embodiment, the expression level of Lamin A increases to between about 50% and about 150% of the wild-type expression level. In another embodiment, the expression level of Lamin A increases to between about 50% and about 200% of the wild-type expression level. In another embodiment, the expression level of Lamin A increases to between about 50% and about 250% of the wild-type expression level. In another embodiment, the expression level of Lamin A increases to between about 100% and about 150% of the wild-type expression level. In another embodiment, the expression level of Lamin A increases to between about 100% and about 200% of the wild-type expression level. In another embodiment, the expression level of Lamin A increases to between about 100% and about 250% of the wild-type expression level. In another embodiment, the expression level of Lamin A increases to between about 100% and about 300% of the wild-type expression level.

In one embodiment, the expression level is determined on the mRNA level. In another embodiment, the expression level is determined on the protein level.

"Wild-type", "control" or "reference" gene expression, mRNA or protein levels are determined by a control sample, cell or organisms, or by averaging the expression levels from multiple control samples, cells or organisms. In the context of the present invention, the term "wild-type" or "control" refers to a cell or organism that is healthy or a sample from a subject that is healthy or to a cell or organism with a specific disease other than laminopathy or a sample from a subject that has been diagnosed with a specific disease other than laminopathy.

In one embodiment, the term "wild-type" when describing Lamin A refers to Lamin A or an isoform of Lamin A, expressed by a wild-type cell or organism or detected in a sample of a healthy subject, without any changes in amino acid sequence. In another embodiment, wild-type Lamin A is encoded by a nucleotide sequence of the *LMNA* gene present in the genome of a wild-type cell or organism or a healthy subject, without any mutations or comprising one or more synonymous/silent mutations.

Functionally, *LMNA* mutation phenotypes can affect the contractile force of cardiomyocytes in subject, increases the risk of beating irregularities (arrhythmia) and increases heart rate in a subject with laminopathy.

The "contractile force" of cardiomyocytes refers to the force that is produced by myosin in muscle cells in order to slide the myofilaments of the cardiac muscle past each other and generate contractions. Reduced contractile force is a common phenotype of cardiomyopathies. Cardiomyocytes need to generate synchronized contractions in order to produce a heartbeat. Contractions are triggered by electrical stimuli in the form of a cardiac action potential, which results in the release of calcium from the sarcoplasmic reticulum, which in turn causes myofibrils to slide past each other in a process called excitation-contraction coupling. Myofibrils are composed of actin filaments and myosin motor proteins, which are organized into sarcomeres and are the contractile units of muscle cells. After hydrolysis of ATP by myosin, force is produced upon sequential conformational changes of the motor triggered by rebinding to F-actin.

Thus, in one embodiment, the expression of Lamin A from the vector increases the absolute force produced by cardiomyocytes affected by laminopathy contacted with the vector by at least 5% compared to cardiomyocytes not contacted with the vector. In another embodiment, the expression of Lamin A from the vector increases the absolute force produced by cardiomyocytes affected by laminopathy contacted with the vector by at least 10%, at least 20%, at least 30%, at least 40%, at least 50%, at least 60%, at least 70%, at least 80%, at least 90%, at least 100%, 110%, at least 120%, at least 130%, at least 140%, at least 150%, at least 160%, at least 170%, at least 180%, at least 190%, or at least 200% compared to cardiomyocytes not contacted with the vector.

In another embodiment, the expression of Lamin A from the vector increases the absolute force produced by cardiomyocytes contacted with the vector by 5% to 200% compared to cardiomyocytes affected by laminopathy not contacted with the vector. In another embodiment, the expression of Lamin A from the vector increases the absolute force produced by cardiomyocytes contacted with the vector by 10% to 200%, by 20% to 200%, by 30% to 200%, by 40% to 200%, by 50% to 200%, by 60% to 200%, by 70% to 200%, by 80% to 200%, by 90% to 200%, by 100 to 200%, 110% to 200%, by 120% to 200%, by 130% to 200%, by 140% to 200%, by 150% to 200%, 10% to 150%, by 20% to 150%, by 30% to 150%, by 40% to 150%, by 50% to 150%, by 60% to 150%, by 70% to 150%, by 80% to 150%, by 90% to 150%, by 100 to 150%, by 10% to 100%, by 20% to 100%, by 30% to 100%, by 40% to 100% or by 50% to 100% compared to cardiomyocytes affected by laminopathy not contacted with the vector.

The term "arrhythmia" as used herein refers to an abnormal heart rhythm. Arrhythmias can be classified by changes in beating rate and beating regularity, and the region of their origin (supraventricular and ventricular; e.g. sinoatrial node, atria, AV node and ventricle). The normal beating activity of the heart origins in the sinoatrial node (called sinus rhythm). The heart can beat in regular sinus rhythm that is too fast (sinus tachycardia) or too slow (sinus bradycardia). Tachycardia is defined as a resting heart rate greater than 100 beats per minute (bpm) while bradycardia is defined as a resting heart rate of less than 60 bpm. The beating activity of the heart can also come from another site than the sinoatrial node, which is generally pathological. Examples are the atria (atrial arrhythmia), the AV node (junctional arrhythmia) and the ventricle (ventricular arrhythmia). Generally, supraventricular arrhythmias are less dangerous than ventricular arrhythmias because the AV node normally filters fast electrical activity from the atria and therefore does not allow the ventricle to beat too fast. Some examples of arrhythmia include sick sinus syndrome, conduction block (leading to bradycardia) and premature heartbeats (can be both supraventricular and ventricular), atrial fibrillation, atrial flutter, supraventricular tachycardia, ventricular tachycardia, ventricular fibrillation (leading to tachycardic disturbances). Ventricular fibrillation is the most severe arrhythmia and, without intervention, leads to death in minutes.

In one embodiment, the presence of arrhythmia in cardiomyocytes affected by laminopathy is detected by determining the variability of beat-to-beat interval (RR scatter).

"Heart rate variability" or abnormal heart rhythm is measured by measuring the variation in the beat-to-beat interval of the heart beat with the help of an electrocardiogram (ECG). Heart rate variability is also called "cycle length variability" or "R-R variability". Herein, "R" refers to the point of the peak of the QRS complex of the electrocardiogram (ECG) wave, and the "RR interval" is the interval between successive Rs. The RR interval is also sometimes called NN interval to indicate that the heart beat is "normal". The "QRS complex" is a combination of three graphical deflections seen on a typical ECG, wherein the "R" deflection is an upward peak or "wave" located between the Q and S downward deflections. Methods used to detect heart beats are known in the art and include ECG, blood pressure, ballistocardiograms and the pulse wave signal derived from a photoplethysmograph (PPG).

In one embodiment, expression of Lamin A from the vector reduces the degree of beating irregularities by at least 5% in cardiomyocytes affected by laminopathy contacted with the vector compared to cardiomyocytes affected by laminopathy not contacted with the vector, wherein the beating irregularities are detected by measuring cycle length variability based on RR intervals.

In another embodiment, expression of Lamin A from the vector reduces the degree of beating irregularities by at least 10%, at least 15%, at least 20%, at least 25%, at least 30%, at least 35%, at least 40%, at least 45%, at least 50%, at least 55%, at least 60%, at least 65%, at least 70%, at least 75% or at least 80% in cardiomyocytes affected by laminopathy contacted with the vector compared to cardiomyocytes affected by laminopathy not contacted with the vector, wherein the beating irregularities are detected by measuring cycle length variability based on RR intervals.

Methods of measuring cycle length variability using RR intervals are known in the art. The most commonly used method is a time-domain method called "SDNN" or standard deviation of NN/RR intervals. This method calculates the standard deviation of the average RR intervals calculated over a 24 hour period, or over a shorter period, in order to represent total variability in the heart rate cycle. Another common time-domain method is the root mean square of successive differences or "RMSSD". This method calculates the square root of the mean of the squares of successive differences in RR intervals. "NN50" or "NN20" methods analyze the number of pairs of successive NNs/RRs that differ by more than 50 ms or 20 ms, respectively.

Geometric methods can also be used to detect cycle length variability based on RR intervals. An RR scatter plot may be used, also termed "Poincaré plot". The Poincaré plot plots RR interval 1 against RR interval 2, RR interval 2 against RR interval 3, and so on. In short, the X-axis of the scatter plot shows $RR_n$ (ms) and the Y-axis of the scatter plot shows $RR_{n+1}$ (ms). Hence, the plot shows how well each RR interval predicts the next, i.e. the variability between one RR interval and the next. A greater spread or scatter of values indicates an increased heart rate variability.

In one embodiment, measuring cycle length variability based on RR intervals means measuring cycle length variability using RR scatter.

Hence, in one embodiment, expression of Lamin A from the vector reduces the degree of beating irregularities by at least 5 % in cardiomyocytes affected by laminopathy contacted with the vector compared to cardiomyocytes affected by laminopathy not contacted with the vector, wherein the beating irregularities are detected by measuring cycle length variability using RR scatter.

In another embodiment, expression of Lamin A from the vector reduces the degree of beating irregularities by at least 10 %, at least 15%, at least 20%, at least 25%, at least 30%, at least 35%, at least 40%, at least 45%, at least 50%, at least 55%, at least 60%, at least 65%, at least 70%, at least 75% or at least 80% in cardiomyocytes affected by laminopathy contacted with the vector compared to cardiomyocytes affected by laminopathy not contacted with the vector, wherein the beating irregularities are detected by measuring cycle length variability using RR scatter.

In another embodiment, expression of Lamin A from the vector reduces beats per minute by at least 1% in cardiomyocytes affected by laminopathy contacted with the vector compared to cardiomyocytes affected by laminopathy not contacted with the vector.

In another embodiment, expression of Lamin A from the vector reduces beats per minute by at least 2%, at least 3%, at least 4%, at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%, at least 21%, at least 22%, at least 23%, at least 24%, at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 44%, at least 45%, at least 46%, at least 47%, at least 48%, at least 49%, or at least 50% in cardiomyocytes affected by laminopathy contacted with the vector compared to cardiomyocytes affected by laminopathy not contacted with the vector.

In one embodiment, expression of Lamin A from the vector for use of the invention reduces the degree of beating irregularities by at least 5 % and/or beats per minute by at least 1% in cardiomyocytes affected by laminopathy contacted with the vector compared to cardiomyocytes affected by laminopathy not contacted with the vector, wherein the beating irregularities are detected by measuring cycle length variability using RR scatter.

**Delivery of gene replacement therapy into cells**

The nucleic acid molecule or the vector of the invention can be delivered by various delivery systems such as adeno-associated viruses (AAV), lentiviruses, adenoviruses, and other viral vectors, or methods, such as transfection, transduction, infection, gene transfer, gene editing, nucleofection or electroporation.

In certain embodiments, the delivery is via adeno-associated viruses (AAV). Adeno-associated viruses are replication-defective, nonenveloped viruses with a linear single-stranded DNA genome. Gene therapy vectors using AAV can infect cells without integration into the host cell genome. At least eleven serotypes of AAV have been identified so far, most of which can infect cells from multiple tissue types. Tissue specificity is determined by the capsid serotype and gene therapy vectors are selected according to their tropism. While the most commonly used serotype is AAV2, the optimal serotype for muscle cell infections are AAV1, AAV6, AAV7, AAV8, and AAV9.

In some embodiments, the delivery is via a recombinant adeno-associated virus (rAAV) vector. For example, in some embodiments, a modified AAV vector may be used for delivery. Modified AAV vectors can be based on one or more of several capsid types, including AAV1, AV2, AAV5, AAV6, AAV8, AAV8.2. AAV9, AAV rhIO, modified AAV vectors (e.g., modified AAV2, modified AAV3, modified AAV6) and pseudotyped AAV (e.g., AAV2/8, AAV2/5 and AAV2/6).

Exemplary AAV vectors and techniques that may be used to produce rAAV particles are known in the art (see, e.g., Aponte-Ubillus et al. Appl. Microbiol. Biotechnol. 2018, PMID: 29204900; Zhong et al. J. Genet. Syndr. Gene Ther. 2012, PMID: 23264889).

Hence, a further aspect of the invention relates to an AAV for use in the treatment of laminopathy in a subject comprising or the nucleic acid molecule for use of the invention or the vector for use of the invention. In one embodiment, the AAV is an AAV with a serotype selected from the group consisting of serotype AAV1, AAV2, AAV6, AAV7, AAV8, and AAV9. Preferably, the AAV is serotype AAV6 or AAV9.

Preferably, the AAV comprises an expression cassette comprising a promoter and a nucleic acid molecule of the invention, encoding Lamin A.

In one embodiment, the AAV comprises a nucleic acid molecule encoding an amino acid sequence that is at least 70% identical to the amino acid sequence according to SEQ ID NO: 1. In one embodiment, the AAV comprises a nucleic acid molecule encoding an amino acid sequence that is at least 75%, at least 80%, at least 85%, at least 90%, at least 95%, at least 98%, at least 99% or 100% identical to the amino acid sequence according to SEQ ID NO: 1.

In another embodiment, the AAV comprises a nucleic acid molecule encoding an amino acid sequence that is at least 70% identical to the amino acid sequence according to SEQ ID NO: 2. In one embodiment, the AAV comprises a nucleic acid molecule encoding an amino acid sequence that is at least 75%, at least 80%, at least 85%, at least 90%, at least 95%, at least 98%, at least 99% or 100% identical to the amino acid sequence according to SEQ ID NO: 2.

In another embodiment, the AAV comprises a nucleic acid molecule comprising a nucleic acid sequence that is at least 70% identical to the nucleic acid sequence according to SEQ ID NO: 3. In another embodiment, the AAV comprises a nucleic acid molecule comprising a nucleic acid sequence that is at least 75%, at least 80%, at least 85%, at least 90%, at least 95%, at least 98%, at least 99% or 100% identical to the nucleic acid sequence according to SEQ ID NO: 3. In another embodiment, the AAV comprises a nucleic acid molecule comprising a nucleic acid sequence according to SEQ ID NO: 3.

The AAV may comprise a nucleic acid molecule encoding any wild-type isoform of Lamin A. Thus, in some embodiments, the AAV comprises a nucleic acid molecule encoding an amino acid sequence that is at least 70% identical to the amino acid sequence according to any one of SEQ ID NOs: 4 - 8. In one embodiment, the AAV comprises a nucleic acid molecule encoding an amino acid sequence that is at least 75%, at least 80%, at least 85%, at least 90%, at least 95%, at least 98%, at least 99% or 100% identical to the amino acid sequence according to any one of SEQ ID NOs: 4 - 8. In one embodiment, the AAV comprises a nucleic acid molecule encoding an amino acid sequence according to any one of SEQ ID NOs: 4 - 8.

In one embodiment, the AAV comprises a nucleic acid molecule encoding an amino acid sequence according to any one of SEQ ID NOs: 1, 2, 4, 5, 6, 7 or 8, preferably wherein the AAV is serotype AAV6 or AAV9.

In one embodiment, the AAV for use is administered at a titer suitable to induce expression of Lamin A to at least 50% of wild-type expression level. In another embodiment, the AAV for use is administered at a titer suitable to induce expression of Lamin A to at least 60%, at least 70%, at least 80%, at least 90%, at least 100%, at least 110%, at least 120%, at least 130%, at least 140%, at least 150%, at least 160%, at least 170%, at least 180%, at least 190%, at least 200%, at least 210%, at least 220%, at least 230%, at least 240%, at least 250%, at least 260%, at least 270%, at least 280%, at least 290%, or at least 300%, of the wild-type expression level.

In another embodiment, the AAV for use is administered at a titer suitable to induce expression of Lamin A to between about 50% and about 300% of the wild-type expression level.

In another embodiment, the AAV for use is administered at a titer suitable to induce expression of Lamin A to between about 50% and about 150% of the wild-type expression level. In another embodiment, the AAV for use is administered at a titer suitable to induce expression of Lamin A to between about 50% and about 200% of the wild-type expression level. In another embodiment, the AAV for use is administered at a titer suitable to induce expression of Lamin A to between about 50% and about 250% of the wild-type expression level. In another embodiment, the AAV for use is administered at a titer suitable to induce expression of Lamin A to between about 100% and about 150% of the wild-type expression level. In another embodiment, the AAV for use is administered at a titer suitable to induce expression of Lamin A to between about 100% and about 200% of the wild-type expression level. In another embodiment, the AAV for use is administered at a titer suitable to induce expression of Lamin A to between about 100% and about 250% of the wild-type expression level. In another embodiment, the AAV for use is administered at a titer suitable to induce expression of Lamin A to between about 100% and about 300% of the wild-type expression level.

In one embodiment, the expression level is determined on the mRNA level. In another embodiment, the expression level is determined on the protein level.

The term "virus particles" or "virus particle units" as used herein refers to a complete virus particle, also known as virion, comprising a nucleic acid surrounded by a protective coat of protein called a capsid.

In one embodiment, the AAV for use is administered at a titer resulting in about 10,000 to about 500,000 virus particles/cell, preferably wherein the cell is a cardiomyocyte. In another embodiment, the AAV for use is administered at a titer resulting in about 100,000 to about 500,000 virus particles/cell, preferably wherein the cell is a cardiomyocyte.

The AAV can be administered in a single dose containing at least 1 x 10$^{10}$ virus particles of adeno-associated viruses per kg of body weight of the subject. In one embodiment, the AAV can be administered in a single dose containing at least 1 x 10$^{12}$ virus particles of adeno-associated viruses per kg of body weight of the subject.

In one embodiment, the AAV is administered to a subject at a dose between to 1x 10$^{12}$ to 2 x 10$^{14}$ virus particles per kg body weight. In one embodiment, the AAV is administered to a subject at a dose of at least about 1 x 10$^{12}$, at least about 2 x 10$^{12}$, at least about 5 x 10$^{12}$, at least about 8 x 10$^{12}$, at least about 1 x 10$^{13}$, at least about 2 x 10$^{13}$, at least about 5 x 10$^{13}$, at least about 8 x 10$^{13}$, at least about 1 x 10$^{14}$ or at least about 2 x 10$^{14}$ virus particles per kg body weight.

In one embodiment, the AAV for use in the treatment of laminopathy in a subject comprising the nucleic acid molecule for use of the invention or the vector for use of the invention, is administered at a titer suitable to induce expression of Lamin A to at least 50% of WT expression, and/or wherein the AAV for use is administered at a titer about 10,000 to about 500,000 virus particles/cell, preferably wherein the AAV is serotype AAV6 or AAV9, optionally wherein the cell is a cardiomyocyte.

In one embodiment, the invention relates to a method of treatment of laminopathy in a human or non-human subject in need thereof comprising administering to said subject a nucleic acid encoding a Lamin A polypeptide, a vector comprising a nucleic acid encoding a Lamin A polypeptide or an AAV comprising a nucleic acid encoding a Lamin A polypeptide. In one embodiment, the laminopathy is dilated cardiomyopathy type 1A.

In one embodiment, the invention relates to the use of a nucleic acid encoding a Lamin A polypeptide, a vector comprising a nucleic acid encoding a Lamin A polypeptide or an AAV comprising a nucleic acid encoding a Lamin A polypeptide in the manufacture of a medicament.

In one embodiment, the invention relates to the use of a nucleic acid encoding a Lamin A polypeptide, a vector comprising a nucleic acid encoding a Lamin A polypeptide or an AAV comprising a nucleic acid encoding a Lamin A polypeptide in the manufacture of a medicament for the treatment of laminopathy. In one embodiment, the laminopathy is dilated cardiomyopathy type 1A.

In a further aspect, the invention also relates to a pharmaceutical composition comprising the nucleic acid molecule encoding Lamin A for use, the vector comprising a nucleic acid molecule encoding Lamin A for use or an AAV comprising a nucleic acid encoding Lamin A for use, and optionally a pharmaceutically acceptable carrier.

In one embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations, and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in Remington's Pharmaceutical Sciences, 18th Ed., Gennaro, ed. (Mack Publishing Co., 1990). In one embodiment, other ingredients can be added to pharmaceutical formulations, including antioxidants, e.g., ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; and sugar alcohols such as mannitol or sorbitol.

The compositions of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropyLamine, triethyLamine, 2-ethyLaminoethanol, histidine, procaine.

In one embodiment, the nucleic acid molecule for use of the invention, the vector for use of the invention, the AAV for use of the invention or the pharmaceutical composition of the invention are administered locally. Preferably, the nucleic acid molecule for use of the invention, the vector for use of the invention, the AAV for use of the invention or the pharmaceutical composition of the invention are administered to muscle tissue. More preferably, the nucleic acid molecule for use of the invention, the vector for use of the invention, the AAV for use of the invention or the pharmaceutical composition of the invention are administered to heart muscle tissue. In one embodiment, the nucleic acid molecule for use of the invention, the vector for use of the invention, the AAV for use of the invention or the pharmaceutical composition of the invention may be administered to heart muscle tissue by cardiac transfusion. Cardiac transfusion may be achieved by enabling direct access to the cardiac muscle.

In another embodiment, the nucleic acid molecule for use of the invention, the vector for use of the invention, the AAV for use of the invention or the pharmaceutical composition of the invention are administered systemically. Systemic routes of administration may include inhalation, injection or infusion. Systemic administration refers to a route of administration that affects the entire body, usually by administration into the circulatory system. Administration may be via injection, preferably via intravenous, intracardiac or intramuscular injection.

Furthermore, the inventors performed a proteome analysis of hiPSC-derived *LMNA* knock-out engineered heart tissue (EHT). They found that expression of Lamin A from the vector of the invention was able to restore expression of cardiac and nuclear Lamina proteins including Lamin B1, Lamin B2, Emerin and SUN domain-containing protein 1.

Hence, in one embodiment, treatment with the nucleic acid for use of the invention, the vector for use of the invention, the AAV for use of the invention or the pharmaceutical composition of the invention may lead to an up-regulation of proteins involved in cardiac contraction. In another embodiment, treatment with the nucleic acid for use of the invention, the vector for use of the invention, the AAV for use of the invention or the pharmaceutical composition of the invention may lead to an up-regulation of one or more proteins selected from Dystrophin, muscular *LMNA*-interacting protein, Cofilin 2, LIM domain -binding protein 3, Lamin B2, Lamin B1, dual specificity phosphatase 3, four and a half LIM domains protein 1, PDZ and LIM domain protein 1, LIM only protein 7, Actin-binding LIM protein 1, Emerin, SUN domain-containing protein 1, PDZ and LIM domain protein 3, $Na^+/K^+$-ATPase subunit $\beta1$, $Na^+/K^+$-ATPase subunit $\alpha1$, Troponin I, Tubulin $\beta$-3 chain, Myosin-binding protein C, Caveolin 1, Titin, Myosin 7, Troponin C, Plakophilin 2, A-actin 1, Junction Plakoglobin, Desmoplakin, Desmoglein 2, Catenin $\alpha3$, Ryanodine receptor 2, and Desmin. Preferably, treatment with the nucleic acid for use of the invention or the vector for use of the invention leads to an up-regulation of one or more proteins selected from Lamin B1, Lamin B2, Emerin and SUN domain-containing protein 1.

## Examples

## Materials and Methods

### *Reprogramming of patient-specific induced pluripotent stem cells*

Fibroblasts from a skin biopsy of the laminopathy patient carrying a heterozygous missense mutation p.H222P in the gene *LMNA* were reprogrammed at passage 7 according to manufacturer's protocol of CytoTune-iPS Sendai Reprogramming Kit (Product No. A1377801, Life Technologies).

### *Generation of a knockout and an isogenic control cell line*

To generate an isogenic control (IsoC), the hetH222P patient cell line was expanded in FTDA media and grown until 80-90% confluency. Cells were pre-incubated with Y-27632 (10 $\mu$M) and bFGF (30 ng/mL) FTDA for one hour. HiPSCs were dissociated with Accutase® into single cells (10 min, 37 °C, 5% $CO^2$, 20% $O^2$). 800,000 cells were resuspended in 100 $\mu$L Nucleofector solution (82 $\mu$L P3 Amaxa Solution and 18 $\mu$L Supplement) and 4 $\mu$L of ssODN (4 $\mu$M) produced by IDT (5'-tctgtgtccttcctccaacccttccagGAGCTGCGTGAGACCAAGCGACGTCATGAGAC-CCGACTGGTGGAGATT GACAATGGGAAGCAGCCGTGAGTTTGAGAGCCGGCTGGC-3' (SEQ ID NO: 9) and 1 $\mu$L RNP complex, Cas9 3NLS/sgRNA complex (5'- CCACCAGTCGGGTCTCAGGA -3' (SEQ ID NO: 10) were added before transferring the cells to the Amaxa nucleofection cassette following manufacturer's instructions (program CA137) for the 4D-NucleofectorTM (Lonza). Following nucleofection hiPSCs were seeded on Matrigel (1:60; Corning®, #354230) coated 12-well and cultured in condition medium (CM; murine embryonic fibroblasts) supplemented with Y-27632 (Biorbyt, orb154626; 10 $\mu$M) and bFGF (30 ng/mL). After 48 hours cells were dissociated and seeded to single cells 200, 400 and 600 cells/per 6-well format. Single clones were cultured on Matrigel coated wells and in CM until they grew in 48-well format followed by cryo preservation. DNA was isolated from 48-well copy plates for genomic analysis via PCR to assess editing efficiency and excluding 10 most probable off-target according to http://crispor.tefor.net/. The clones which were chosen were analysed for karyotype using the NanoString nCounter® Human Karyotype Panel according to manufacturer's protocol.

### *HiPSC expansion, cardiac differentiation and EHT generation*

HiPSCs were cultured and expanded with a chemically defined media and quality controls (pluripotency marker SSEA3 and contamination tests) were carried out according to Shibamiya et al. 2020. Cardiac differentiation and dissociation was achieved in a 2D monolayer protocol from Mosquiera et al. 2018 with an adapted seeding density hiPSCs of 50,000-60,000 cells/cm$^2$. Dissociated cardiomyocytes were counted and analysed for differentiation efficiency as recently described with an intracellular cardiac troponin T (cTnT) staining via flow cytometry. Cell preparations with a cTnT positivity above 80% were subjected to EHT generation as previously described (Breckwoldt et al. 2017, Nat. Protoc.; Mannhardt et al. 2016 Stem Cell Reports 7:29-42) with an adapted master mix containing only DMEM medium (PAN BioTech, P04-04510), supplemented with 10% heat-inactivated horse serum (Thermo Fisher. #26050088), 1% penicillin/ streptomycin (PEST, Gibco), 1X GlutaMAX™ (Gibco, 35050038), Y-27632 and 5mg/ml fibrinogen (Sigma #F8630).

### rAA V Vector particle production and purification

Adeno-associated virus (AAV) vector particles were produced as recently published with some adaptions (Saleem et al. 2020 Stem Cell Reports 14: 312-324). GFP and Lamin A cDNA (*LMNA*) were assembled via a 2A peptide and inserted into pFBGR under control of a CMV enhanced promoter by InFusion cloning. Two fragments were generated by PCR using PrimeStar GLX Polymerase (Clontech): GFP was amplified from pCW57-GFP-2A-MCS (Addgene Plasmid #71783) using 5'-GTCAGATCCGCTAGCGCTaccATGGTGAGCAAGGGC (SEQ ID NO: 11) and 5'-gtctccatGCTAGCcggTGCag (SEQ ID NO: 12). *LMNA* was amplified from PA-mCherry1-LaminA-C-18 (Addgene, plasmid #57185) using 5'-gGCTAGCatggagacccgtccc (SEQ ID NO: 13) and 5'-tatagggcgaattgggtaccttacatgatgctgcagttctgggg (SEQ ID NO: 14). pFBGR-Ultra CMVenh-GFP was cut with AfeI and KpnI and both fragments were inserted simultaneously using the InFusion HD Cloning Kit (Takara Clontech) according to the recommendations of the manufacturer to generate the AAV transfer plasmid pFBGR CMVenh GFP-2A-*LMNA*. Final AAV transfer plasmids were confirmed by restriction digest, by PCR using the specific primers mentioned above and by sequencing.

### EHT culture and AAV Transduction

EHTs were cultured in "complete medium" DMEM medium (PAN BioTech, P04-04510, supplemented with 10% heat-inactivated horse serum [Thermo Fisher, #26050088], 1% penicillin/streptomycin [PEST, Gibco], 0.1% insulin [Sigma Aldrich, I9278] and 0.1% aprotinin [Sigma Aldrich, A1163]). The medium was changed every two to three days and the force development was analysed with the equipment (A003 and A004), which was purchased from EHT Technologies GmbH (Hamburg, Germany). On day 15 of culture, the gene therapy groups were transduced with the AAV6-CMV-GFP-2A-c*LMNA* virus (MOI of 30,000 or 60,000) adding directly to the complete media and shaking for 72 hours (37 °C, 7% CO$^2$, 40% O$^2$).

### Western blot analysis

Liquid nitrogen frozen EHTs were thawed, and protein was extracted with TRIzol® Reagent according to manufacturer's protocol. Protein pellet was resuspended in 100 μL Kranias buffer and 20 uL 6x Laemmli Buffer and boiled at 95 °C for 5 minutes after a short spin down 12 uL of sample were loaded onto a 4-15% Miniprotean TGX gel (BioRad; #4561083) and run at 80 V for 10 minutes following 110 V for 100 minutes. Protein transfer occurred at 300 mA for 90 minutes on a nitrocellulose membrane. The membrane was blocked in 5% milk TBS-T and stained with anti-Lamin A/C (Santa Cruz, sc-376248 E-1; 1:500), anti-MYBPC3 (Santa Cruz, sc-137181, F-1, 1:1,000), anti-GFP (Abcam, ab290; 1:2,000). Secondary antibodies conjugated with peroxidase directed against mouse (Sigma Aldrich, A9044; 1:6,000) and rabbit (Sigma Aldrich A0545; 1:5,000) were used for secondary staining. And using the Clarity™ Western ECL Substrate (Bio-Rad, #1705061) on a ChemiDoc™ Touch Imaging System (Bio-Rad) Signals were detected and quantified using the Image Lab™ 5.2.1 software (Bio-Rad).

### Immunofluorescence of engineered heart tissue

EHTs were washed two times in 1x PBS and fixed overnight in 1.5 mL Histofix® (Carl Roth) at 4 °C. Next day, EHTs were transferred to TBS-azide. EHTs were blocked in blocking solution (1X PBS [Gibco], 3% w/v milk powder [Carl Roth], 0.1% Triton X-100 [Roth 3051.3]) agitating at 4 °C overnight. After washing three times, tissues in 1x PBS EHTs were stained in blocking solution with anti-Lamin A/C (Santa Cruz, sc-376248, E-1; 1:500), anti-alpha-actinin2 (Sigma Aldrich, SAB2108642; 1:800) and Hoechst (Thermo Fisher Scientific, 33352; 1:2,000) overnight agitating at 4 °C. Subsequent to another washing procedure, EHTs were incubated wit secondary antibodies anti-mouse Alexa Fluor 633 (Life Technologies, 1:800) and anti-rabbit Alexa Fluor 546 (Life Technologies, 1:800). Following a final washing procedure EHTs were mounted onto cover slips and analysed by confocal microscopy (Zeiss LSM 800 microscope). Images were processed with the ZEN (2012) Blue Edition software.

## 2D Cardiomyocyte culture and immunofluorescence

CMs were seeded as single cells as described previously with 5,000 and 10,000 cells per well.[25] CMs were cultured as EHTs only with 100 uL complete media and transduced as EHTs on day 15 with an MOI of 30,000 but centrifuged with 2500 rpm at 30 °C for 30 minutes. After 28 days in culture CMs were prepared for immunofluorescence as according to Prondzynski et al., but were stained with anti-Emerin (Novocastra, 4G5; 1:100), anti-alpha-actinin2 (Sigma Aldrich, SAB2108642; 1:800) and Hoechst 33258 (Thermo Fisher, #33258;).

## RNA Seq Analysis

RNA of snap frozen EHTs were isolated following the TRIzol manufacturer's protocol (Thermofisher, 15596026). Sequencing was performed at the Core Facility Genomics of the Medical Faculty Münster. Quality and integrity of total RNA was controlled on Agilent Technologies 2100 Bioanalyzer (Agilent Technologies; Waldbronn, Germany). RNA was purified from 150 ng total RNA using the NEBNext rRNA Depletion Kit (NEB E6310, New England Biolabs). The RNA Sequencing library was prepared with NEBNext® Ultra™ II Directional RNA Library Prep Kit for Illumina® (New England Biolabs). The libraries were sequenced on Illumina Nextseq 2000 using NextSeq2000 P3 Reagent Kit (200 cycles, paired end run 2x 111 bp) with an average of 37.9 M reads per RNA sample. Using a molecular barcode, the samples were demultiplexed (bcl2fastq2) to fastq data and quality controlled (FastQC). Trimmomatic was used for adapter trimming and read filtering (Bolger et al., Bioinformatics 2014, PMID: 24695404). The resulting reads were aligned to the Ensembl GRCh38 reference genome using HISAT2 (Kim et al, Nat Methods 2015, PMID: 25751142).
The aligned reads were sorted using samtools (Li et al, Bioinformatics 2011, PMID: 21903627).
The sorted and aligned reads were counted into genes using HTSeq-Counts (Anders et al., Bioinformatics 2015, PMID: 25260700). The test for differential expression were performed
using the r-package DESeq2 (Love et al., Genome Biol. 2014, PMID: 25516281). The GSEA software, a joint project of UC San Diego and Broad Institute was used for the Gene Set Enrichment Analysis with the Gene Ontology gene sets c5.go.v7.4.symbols from the Broad Institute (Goswami et al, Front. Oncol. 2019, PMID: 31069169; Subramanian et al., Proc. Natl. Acad. Sci. 2005, PMID: 16199517).

## Cell lysis and preparation for proteome analysis

EHTs were lysed in 100 mM triethyl ammonium bicarbonate (TEAB) and 1% w/w sodium deoxycholate (SDC) buffer, boiled at 95 °C for 5 min. Samples were sonicated with a probe sonicator for 6 pulses to destroy DNA/RNA. For proteome analysis 20 $\mu$g of protein of each sample was taken and disulfide bonds reduced in the presence of 10 mM dithiothreitol (DTT) at 60 °C for 30 min. Cysteine residues were alkylated in presence of 20 mM iodoacetamide at 37 °C in the dark for 30 min and tryptic digestion (sequencing grade, Promega) was performed at a 100:1 protein to enzyme ration at 37 °C overnight. Digestion was stopped and SDC precipitated by the addition of 1% v/v formic acid (FA). Samples were centrifuged at 16,000 g for 5 min and the supernatant was transferred into a new tube. Samples were dried in a vacuum centrifuge.

## LC-MS/MS in data dependent mode

Samples were resuspended in 0.1% FA and transferred into a full recovery autosampler vial (Waters). Chromatographic separation was achieved on a Dionex Ultimate 3000 UPLC system (Thermo Fisher Scientific) with a two-buffer system (buffer A: 0.1% FA in water, buffer B: 0.1% FA in ACN). Attached to the UPLC was an Acclaim PepMap 100 C18 trap (100 $\mu$m x 2 cm, 100 A pore size, 5 $\mu$m particle size, Waters) for desalting a purification followed by nanoEase M/Z peptide BEH130 C18 column (75 $\mu$m x 25 cm, 130 A pore size, 1.7 $\mu$m particle size, Waters). Peptides were separation using a 60 min gradient with increasing ACN concentration from 2% - 30% ACN. The eluting peptides were analyzed on a quadrupole orbitrap ion trap tribrid mass spectrometer (Fusion, Thermo Fisher Scientific) in data dependent acquisition (DDA) mode. For DDA analysis, the fusion was operated at top speed mode analyzing the most intense ions per precursor scan (2x105 ions, 120,000 Resolution, 120 ms fill time) within 3 s and were analyzed by MS/MS in the ion trap (HCD at 30 normalized collision energy, 1x104 ions, 60 ms fill time) in a range of 400 - 1300 m/z. A dynamic precursor exclusion of 20 s was used.

## Data analysis and processing

Acquired DDA LC-MS/MS data were searched against the reviewed human protein database downloaded from Uniprot (release April 2020, 20,365 protein entries) and all Lamin A/C proteolytic and splice variants using the Sequest algorithm integrated in the Proteome Discoverer software version 2.4 in label free quantification mode with match between runs enabled, performing chromatographic retention re-calibration for precursors with a 5 min retention time tolerance, no scaling, and no normalisation for extracted peptide areas was done. Mass tolerances for precursors was set to 10 ppm and 0.6 Da for fragments. Carbamidomethylation was set as a fixed modification for cysteine residues and the oxidation of methionine, pyro-glutamate formation at glutamine residues at the peptide N-terminus as well as acetylation of the protein N-terminus, methionine loss at the protein N-terminus and the Acetylation after methionine loss at the protein N-terminus were allowed as variable modifications. Only peptide with a high confidence (false discovery rate < 1% using a decoy data base approach) were accepted as identified. Peptide areas were summed to protein areas and used for quantitative analysis. Protein areas were imported into Perseus software version 1.5.8 for statistical analysis.

**Example 1: Generation of laminopathy patient cell lines and its corresponding isogenic control**

Fibroblasts were isolated from a skin biopsy of a patient with a p.H222P missense mutation in the *LMNA* gene and were reprogrammed to iPSC. CRISPR/Cas9 gene editing was used to produce two cell lines, an isogenic control (IsoC) in which the mutation was corrected and *LMNA* produced wild-type Lamin A and a *LMNA* knock-out cell line homozygous for a truncating mutation at position p.E223X (*LMNA*-KO; overview on experimental strategy in Figure 1). Pluripotency and normal karyotype were confirmed in the cell lines lines. In addition, the two lines generated via CRISPR/Cas9 showed no editing effect in the 10 most likely off-targets.. Cells were differentiated to cardiomyocytes (CMs) and cultured in 2D and 3D format. To explore the effect of gene replacement, CM were transduced with an AAV6 vector encoding a cDNA of *LMNA* and GFP separated by 2A peptide. An exemplary expression vector comprising CMV promoter, the cDNAs of LMNA and GFP separated by a 2A peptide is represented by the nucleic acid sequence according to SEQ ID NO: 19.

**Example 2: Functional phenotype of a laminopathy disease model in EHTs can be rescued by a gene replacement therapy.**

Differentiated CM were analysed in the 3D engineered heart tissues (EHT) culture format to analyse the morphological and functional consequence of the homozygous nonsense mutation of *LMNA*-KO. For each cell line, EHTs were split into two groups that were subjected to AAV6-*LMNA* transduction (gene therapy; GT) or vehicle on day 15. *LMNA*-KO EHTs exhibited lower remodelling capacity, i.e. the reduction in width of the EHT over time (Figure 2 A). The tissues' contractile force development was tracked video-optically for up to 31 days (Figure 2 B). *LMNA*-KO EHTs showed a delayed start of coherent beating and force development compared to IsoC and reached approximately 67% of IsoC at day 19/20. Thereafter, force continuously decreased to a mean of 24% of IsoC on day 28/31 (Figure 2 C and D). AAV6-*LMNA* transduction of *LMNA*-KO EHTs normalized remodelling and prevented the force collapse with mean forces of 52% of IsoC on day 28/31. GT of IsoC had no effect. To better see the effect of *LMNA* overexpression, force at the last day of culture was normalized within each batch to its maximum mean during culture time (Figure 2E). This analysis demonstrated significant stabilisation of force by GT in *LMNA*-KO (78% vs. 40% of maximum)

**Example 3: Gene replacement therapy in isogenic control showed no adverse effects on contractile function**

Given the critical role of Lamin A/C in nuclear function and some evidence from a transgenic mouse model and prior art reports, it is likely that excessive Lamin A expression exerts toxic effects. To assess potential adverse effects of AAV6-*LMNA* on contractile function in IsoC, all functional parameters of a total of 39 EHTs of 4 batches untreated IsoC and 3 batches IsoC GT were normalized to the overall mean. The fold change varied between 0.5 and 2, except for the RR scatter, which indicates an irregular beating pattern and ranged up to 10-fold of the overall mean (Figure 3). No differences were observed between the groups in any of the parameters.

**Example 4: Gene replacement therapy rescues heart beat frequency and variability phenotypes**

Beats per minute (bpm) and RR scatter were analysed in *LMNA*-KO, *LMNA*-KO GT, IsoC and IsoC GT groups. *LMNO*-KO showed increased bpm compared to IsoC line, while gene replacement was able decrease bpm to control levels (Figure 4 A). Degree of heart rate variability was measured by plotting RR-Scatter. *LMNO*-KO showed increased RR-Scatter, i.e. a higher degree of heart rate variability compared to IsoC line, while gene replacement was able decrease the degree of RR-scatter by over 60% to control levels (Figure 4 B). These results show that gene replacement therapy can rescue functional phenotypes of cardiomyocytes affected by laminopathy.

**Example 5: Transcriptome changes in AAV6-*LMNA* treated *LMNA*-KO reflect improved contractile function**

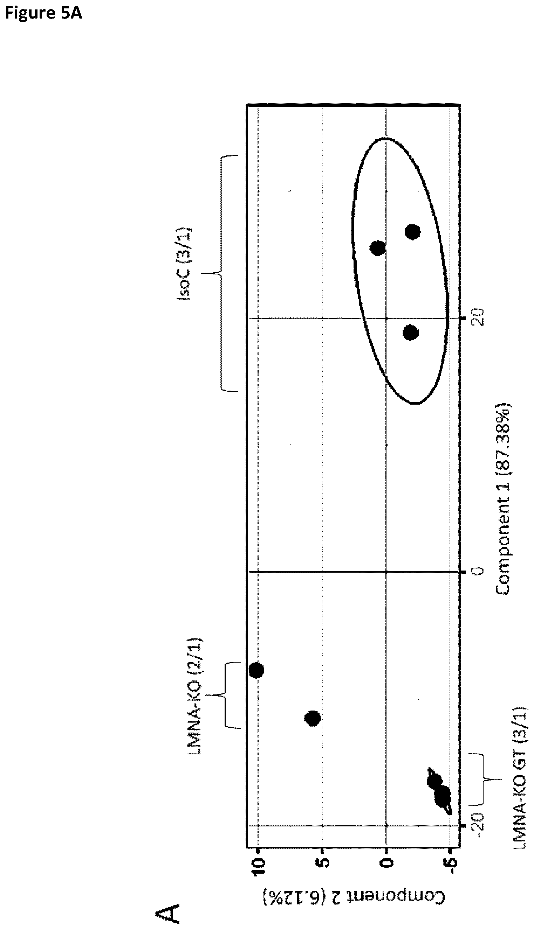

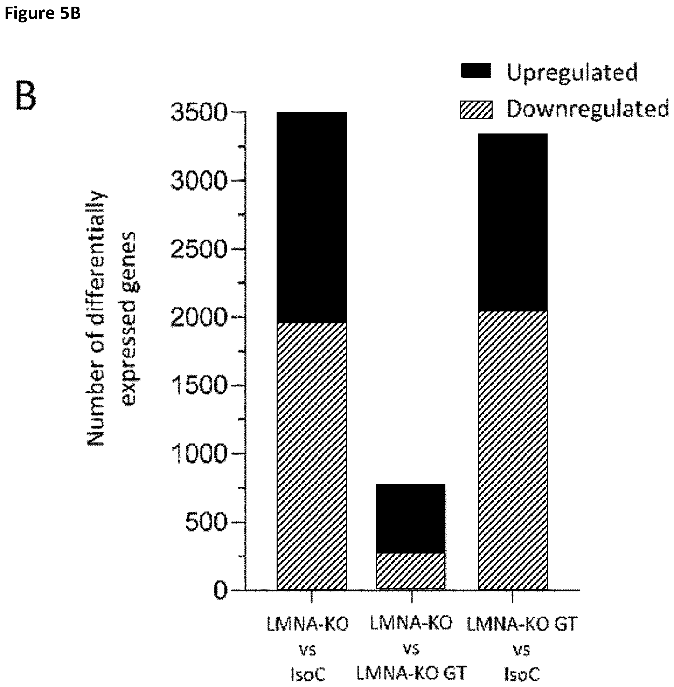

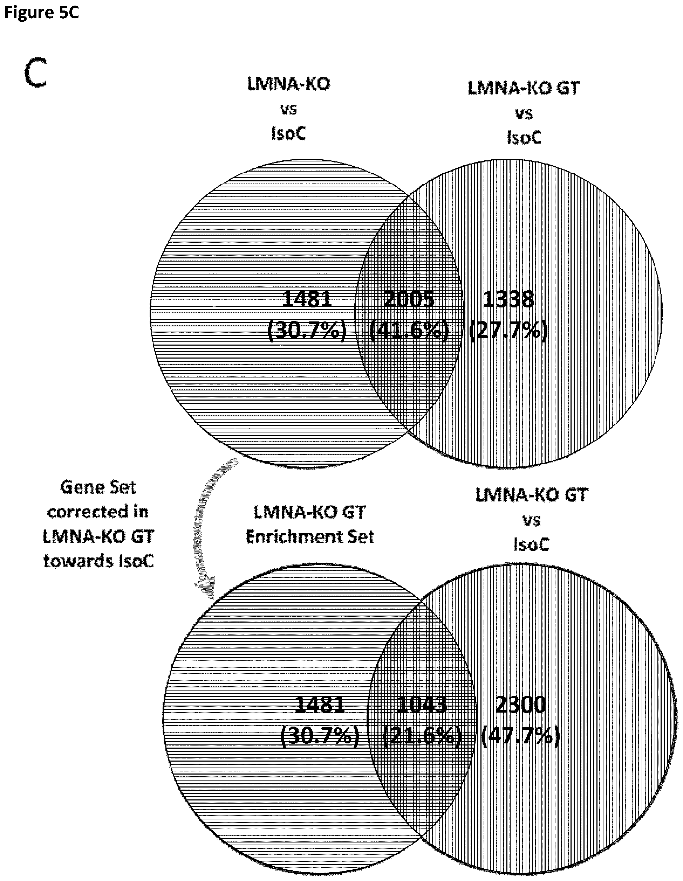

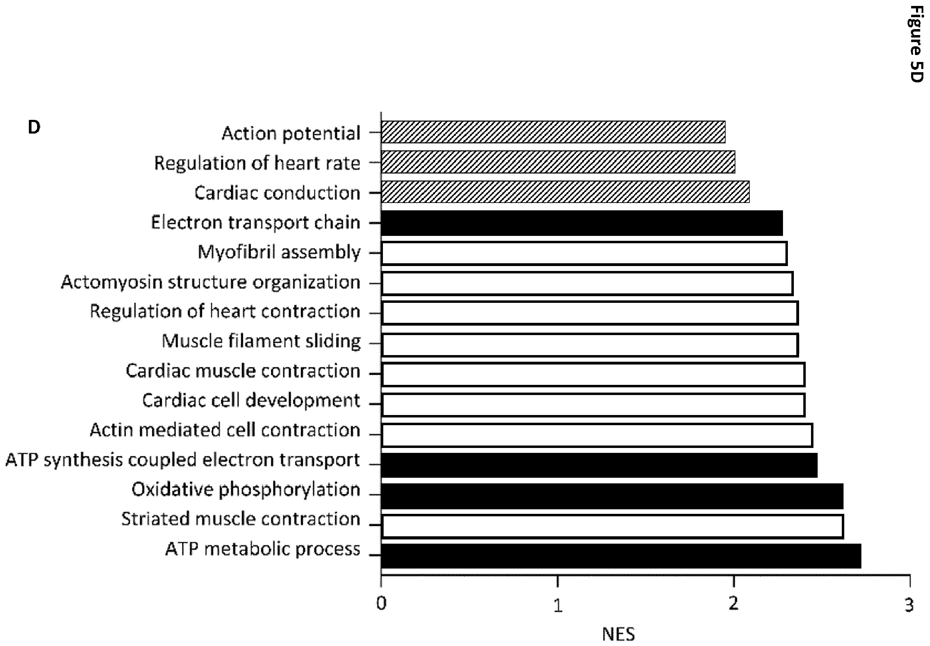

Due to the important role of A-type Lamins in the regulation of gene expression, EHTs were analysed by RNA-sequencing. Principal component analysis revealed distinct clustering of IsoC, *LMNA*-KO and *LMNA*-KO GT EHTs with greatest overall distance between KO and IsoC. *LMNA*-KO GT remained far distant from IsoC in component 1, but moved to IsoC level in component 2 (Figure 5 A). When comparing the number of differentially expressed genes (DEG), *LMNA*-KO and *LMNA*-KO GT showed both more than 3400 DEG versus IsoC (Figure 5 B), whereas only 483 DEG differed between the two groups. The two analyses indicated partial normalisation by gene replacement in *LMNA*-KO. To explore this further, we analysed how many of the 3486 DEG between *LMNA*-KO and IsoC were corrected upon gene therapy towards the values in IsoC (Figure 5 C). Remarkably, this gene set of 2524 DEG when analysed for gene ontology (GO) enrichment falls into 97 significant GO pathways, all of them were upregulated by *LMNA* overexpression, 49 belong to cardiac contraction and development, 24 to mitochondrial respiration and 3 to cardiac electrophysiology (Figure 5 D).

**Example 6: Complete absence of Lamin A and C protein in *LMNA*-KO**

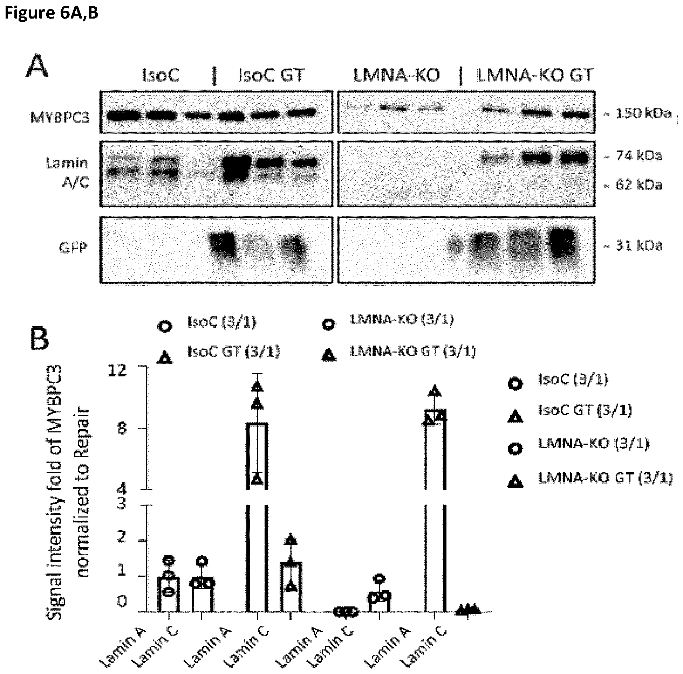

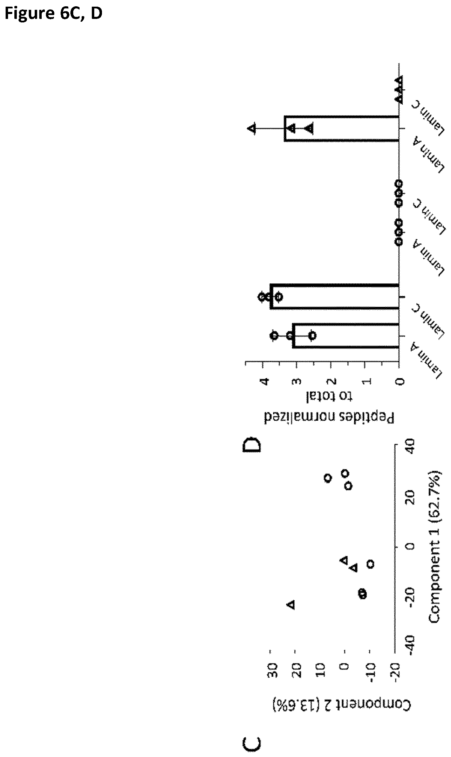

To validate the Lamin knockout and the treatment effect on the protein level, EHT lysates of all four groups were analysed by western blot (Figure 6 A, B). Lamin A and C in IsoC showed the expected two bands at 74 and 70 kDa, respectively. No band was detectable in *LMNA*-KO, indicating complete absence of the proteins. Lamin C was absent in *LMNA*-KO GT and present in IsoC GT. To validate the findings, mass spectrometry (MS) analysis was performed with IsoC, *LMNA*-KO and *LMNA*-KO GT EHTs. No peptides for Lamin A nor C were detected in the *LMNA*-KO group (Figure 6 C). As expected, Lamin C was not present in *LMNA*-KO GT.

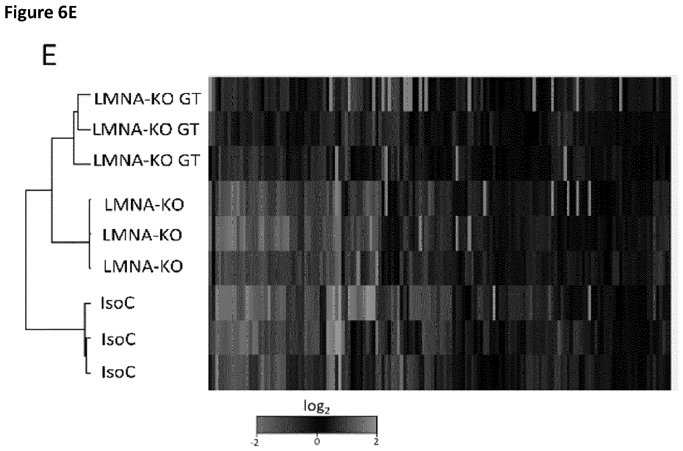

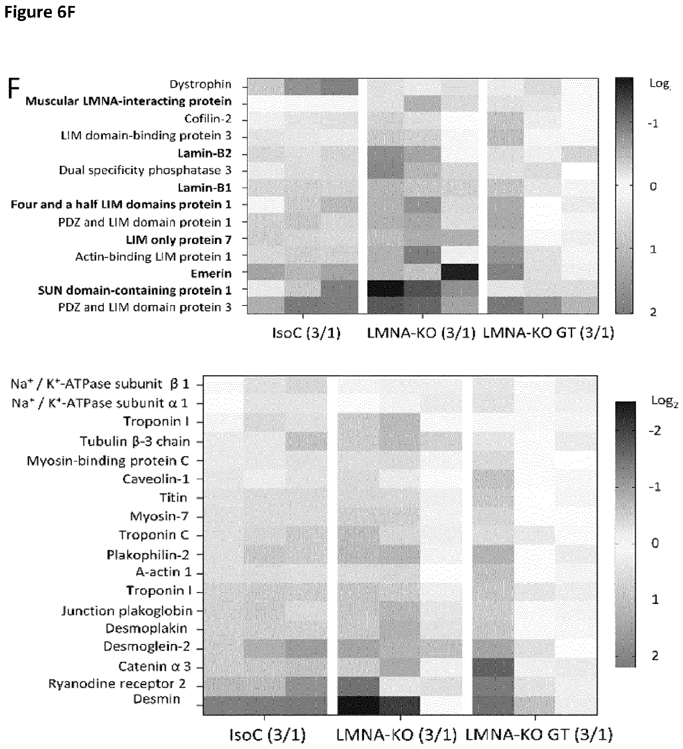

Assessment of the total proteome profile showed a close clustering of *LMNA*-KO and *LMNA*-KO GT and distance of both groups from the IsoC group in a principal component analysis (Figure 6 D). Scattering in *LMNA*-KO GT was high. Supervised clustering of the three groups showed small shifts in *LMNA*-KO GT towards the IsoC proteome profile and away from *LMNA*-KO (Figure 6 E). To investigate this shift further, selected proteins known to be altered in DCM or being in interaction with Lamin A/C were compared (Figure 6 F). Protein levels of e.g. B-type Lamins, emerin and SUN domain-containing protein 1 were lower in *LMNA*-KO EHTs than in IsoC and were partially normalized in *LMNA*-KO GT (Figure 6 F). Proteins involved in cardiac contraction were mostly less abundant in *LMNA*-KO EHT lysates, but partially restored upon gene therapy (Figure 6 F).

**Example 7: Restored remodelling and Lamin A expression and localisation in *LMNA*-KO GT EHTs**

Given *LMNA*-KO EHTs' tendency to lower remodelling capacity, tissues were fixed at the end of the time course experiment (>30 days) and stained for cardiac α-actinin 2 to assess their morphology (Figure 7 A). An overview of stitched confocal images showed a lower density of iPSC-CM and lower sarcomere organisation in *LMNA*-KO than IsoC. *LMNA*-KO EHTs showed more frequently degraded cells in the tissue matrix, indicated by Hoechst-positive nuclear debris (Figure 7 B). Lamin A/C signals were absent in *LMNA*-KO EHTs. The GT groups showed GFP signals homogenously distributed throughout the EHT. The morphological abnormalities observed in *LMNA*-KO were much less apparent in *LMNA*-KO GT EHTs. Expression and nuclear localisation of Lamin A were restored in *LMNA*-KO GT (Figure 7 B). IsoC-GT EHTs showed no systematic differences to IsoC. No mis-localisation or aggregates of Lamin A/C were detected in any group.

**Example 8. Restored Emerin expression and localisation in *LMNA*-KO treated 2D cells**

In line with the lower abundance of Emerin in the MS analysis ($Log_2$Fold of -0.859 versus 1.265), Emerin was undetectable in 2D cultured *LMNA*-KO iPSC-CM and strongly stained in IsoC (Figure 8). *LMNA*-KO GT cells were positive for GFP and showed staining of Emerin at the nuclear envelope, indicating restored Emerin expression and correct localisation.

**Sequences**

**Sequences** included in this description in this section "Sequences" rule over the sequences of the sequence listing according to WIPO ST.25 standard in case there are conflicts.

**SEQ ID NO: 1 - preLamin A wild-type amino acid (Isoform A)**

METPSQRRATRSGAQASSTPLSPTRITRLQEKEDLQELNDRLAVYIDRVRSLETENAGLRLRITESEEVVSREVS
GIKAAYEAELGDARKTLDSVAKERARLQLELSKVREEFKELKARNTKKEGDLIAAQARLKDLEALLNSKEAALS
TALSEKRTLEGELHDLRGQVAKLEAALGEAKKQLQDEMLRRVDAENRLQTMKEELDFQKNIYSEELRETKRR
HETRLVEIDNGKQREFESRLADALQELRAQHEDQVEQYKKELEKTYSAKLDNARQSAERNSNLVGAAHEEL
QQSRIRIDSLSAQLSQLQKQLAAKEAKLRDLEDSLARERDTSRRLLAEKEREMAEMRARMQQQLDEYQELL
DIKLALDMEIHAYRKLLEGEEERLRLSPSPTSQRSRGRASSHSSQTQGGGSVTKKRKLESTESRSSFSQHARTS
GRVAVEEVDEEGKFVRLRNKSNEDQSMGNWQIKRQNGDDPLLTYRFPPKFTLKAGQVVTIWAAGAGATH
SPPTDLVWKAQNTWGCGNSLRTALINSTGEEVAMRKLVRSVTVVEDDEDEDGDDLLHHHHGSHCSSSGD
PAEYNLRSRTVLCGTCGQPADKASASGSGAQVGGPISSGSSASSVTVTRSYRSVGGSGGGSFGDNLVTRSY
LLGNSSPRTQSPQNCSIM

*SEQ ID NO: 2 - mature Lamin A wild-type amino acid*

METPSQRRATRSGAQASSTPLSPTRITRLQEKEDLQELNDRLAVYIDRVRSLETENAGLRLRITESEEVVSREVS
GIKAAYEAELGDARKTLDSVAKERARLQLELSKVREEFKELKARNTKKEGDLIAAQARLKDLEALLNSKEAALS
TALSEKRTLEGELHDLRGQVAKLEAALGEAKKQLQDEMLRRVDAENRLQTMKEELDFQKNIYSEELRETKRR
HETRLVEIDNGKQREFESRLADALQELRAQHEDQVEQYKKELEKTYSAKLDNARQSAERNSNLVGAAHEEL
QQSRIRIDSLSAQLSQLQKQLAAKEAKLRDLEDSLARERDTSRRLLAEKEREMAEMRARMQQQLDEYQELL
DIKLALDMEIHAYRKLLEGEEERLRLSPSPTSQRSRGRASSHSSQTQGGGSVTKKRKLESTESRSSFSQHARTS
GRVAVEEVDEEGKFVRLRNKSNEDQSMGNWQIKRQNGDDPLLTYRFPPKFTLKAGQVVTIWAAGAGATH
SPPTDLVWKAQNTWGCGNSLRTALINSTGEEVAMRKLVRSVTVVEDDEDEDGDDLLHHHHGSHCSSSGD
PAEYNLRSRTVLCGTCGQPADKASASGSGAQVGGPISSGSSASSVTVTRSYRSVGGSGGGSFGDNLVTRSY

*SEQ ID NO: 3 - LMNA wild-type nucleic acid*

atggagaccccgtcccagcggcgcgccacccgcagcggggcgcaggccagctccactccgctgtcgcccacccgcatcacccggct
gcaggagaaggaggacctgcaggagctcaatgatcgcttggcggtctacatcgaccgtgtgcgctcgctggaaacggagaacgca
gggctgcgccttcgcatcaccgagtctgaagaggtggtcagccgcgaggtgtccggcatcaaggccgcctacgaggccgagctcgg
ggatgcccgcaagacccttgactcagtagccaaggagcgcgcccgcctgcagctggagctgagcaaagtgcgtgaggagtttaagg
agctgaaagcgcgcaataccaagaaggagggtgacctgatagctgctcaggctcggctgaaggacctggaggctctgctgaactcc
aaggaggccgcactgagcactgctctcagtgagaagcgcacgctggagggcgagctgcatgatctgcggggccaggtggccaagc
ttgaggcagccctaggtgaggccaagaagcaacttcaggatgagatgctgcggcgggtggatgctgagaacaggctgcagaccat

gaaggaggaactggacttccagaagaacatctacagtgaggagctgcgtgagaccaagcgccgtcatgagacccgactggtggag
attgacaatgggaagcagcgtgagtttgagagccggctggcggatgcgctgcaggaactgcgggcccagcatgaggaccaggtgg
agcagtataagaaggagctggagaagacttattctgccaagctggacaatgccaggcagtctgctgagaggaacagcaacctggtg
gggctgcccacgaggagctgcagcagtcgcgcatccgcatcgacagcctctctgcccagctcagccagctccagaagcagctggca
gccaaggaggcgaagcttcgagacctggaggactcactggcccgtgagcgggacaccagccggcggctgctggcggaaaagga
gcgggagatggccgagatgcgggcaaggatgcagcagcagctggacgagtaccaggagcttctggacatcaagctggccctgga
catggagatccacgcctaccgcaagctcttggagggcgaggaggagaggctacgcctgtcccccagccctacctcgcagcgcagcc
gtggccgtgcttcctctcactcatcccagacacagggtgggggcagcgtcaccaaaaagcgcaaactggagtccactgagagccgca
gcagcttctcacagcacgcacgcactagcgggcgcgtggccgtggaggaggtggatgaggagggcaagtttgtccggctgcgcaa
caagtccaatgaggaccagtccatgggcaattggcagatcaagcgccagaatggagatgatcccttgctgacttaccggttcccaccaa
agttcaccctgaaggctgggcaggtggtgacgatctgggctgcaggagctggggccacccacagcccccctaccgacctggtgtgg
aaggcacagaacacctggggctgcgggaacagcctgcgtacggctctcatcaactccactggggaagaagtggccatgcgcaagct
ggtgcgctcagtgactgtggttgaggacgacgaggatgaggatggagatgacctgctccatcaccaccacggctcccactgcagcag
ctcgggggacccgctgagtacaacctgcgctcgcgcaccgtgctgtgcgggacctgcgggcagcctgccgacaaggcatctgcca
gcggctcaggagcccaggtgggcggacccatctcctctggctcttctgcctccagtgtcacggtcactcgcagctaccgcagtgtgggg
ggcagtggggtgtggcagcttcggggacaatctggtcacccgctcctacctcctgggcaactccagcccccgaacccagagccccag
aactgcagcatcatgtaa

*SEQ ID NO: 4 - Lamin A wild-type isoform C amino acid*

METPSQRRATRSGAQASSTPLSPTRITRLQEKEDLQELNDRLAVYIDRVRSLETENAGLRLRITESEEVVSREVS
GIKAAYEAELGDARKTLDSVAKERARLQLELSKVREEFKELKARNTKKEGDLIAAQARLKDLEALLNSKEAALS
TALSEKRTLEGELHDLRGQVAKLEAALGEAKKQLQDEMLRRVDAENRLQTMKEELDFQKNIYSEELRETKRR
HETRLVEIDNGKQREFESRLADALQELRAQHEDQVEQYKKELEKTYSAKLDNARQSAERNSNLVGAAHEEL
QQSRIRIDSLSAQLSQLQKQLAAKEAKLRDLEDSLARERDTSRRLLAEKEREMAEMRARMQQQLDEYQELL
DIKLALDMEIHAYRKLLEGEEERLRLSPSPTSQRSRGRASSHSSQTQGGGSVTKKRKLESTESRSSFSQHARTS
GRVAVEEVDEEGKFVRLRNKSNEDQSMGNWQIKRQNGDDPLLTYRFPPKFTLKAGQVVTIWAAGAGATH
SPPTDLVWKAQNTWGCGNSLRTALINSTGEEVAMRKLVRSVTVVEDDEDEDGDDLLHHHHVSGSRR

*SEQ ID NO: 5 - Lamin A wild-type isoform ADelta10 amino acid*

METPSQRRATRSGAQASSTPLSPTRITRLQEKEDLQELNDRLAVYIDRVRSLETENAGLRLRITESEEVVSREVS
GIKAAYEAELGDARKTLDSVAKERARLQLELSKVREEFKELKARNTKKEGDLIAAQARLKDLEALLNSKEAALS
TALSEKRTLEGELHDLRGQVAKLEAALGEAKKQLQDEMLRRVDAENRLQTMKEELDFQKNIYSEELRETKRR
HETRLVEIDNGKQREFESRLADALQELRAQHEDQVEQYKKELEKTYSAKLDNARQSAERNSNLVGAAHEEL
QQSRIRIDSLSAQLSQLQKQLAAKEAKLRDLEDSLARERDTSRRLLAEKEREMAEMRARMQQQLDEYQELL
DIKLALDMEIHAYRKLLEGEEERLRLSPSPTSQRSRGRASSHSSQTQGGGSVTKKRKLESTESRSSFSQHARTS
GRVAVEEVDEEGKFVRLRNKSNEDQSMGNWQIKRQNGDDPLLTYRFPPKFTLKAGQVVTIWAAGAGATH
SPPTDLVWKAQNTWGCGNSLRTALINSTGEGSHCSSSGDPAEYNLRSRTVLCGTCGQPADKASASGSGA
QVGGPISSGSSASSVTVTRSYRSVGGSGGGSFGDNLVTRSYLLGNSSPRTQSPQNCSIM

*SEQ ID NO: 6 - Lamin A wild-type isoform 4 amino acid*

MGNSEGCNTKKEGDLIAAQARLKDLEALLNSKEAALSTALSEKRTLEGELHDLRGQVAKLEAALGEAKKQLQ
DEMLRRVDAENRLQTMKEELDFQKNIYSEELRETKRRHETRLVEIDNGKQREFESRLADALQELRAQHEDQV
EQYKKELEKTYSAKLDNARQSAERNSNLVGAAHEELQQSRIRIDSLSAQLSQLQKQLAAKEAKLRDLEDSLA
RERDTSRRLLAEKEREMAEMRARMQQQLDEYQELLDIKLALDMEIHAYRKLLEGEEERLRLSPSPTSQRSRGR
ASSHSSQTQGGGSVTKKRKLESTESRSSFSQHARTSGRVAVEEVDEEGKFVRLRNKSNEDQSMGNWQIKR
QNGDDPLLTYRFPPKFTLKAGQVVTIWAAGAGATHSPPTDLVWKAQNTWGCGNSLRTALINSTGEEVAM
RKLVRSVTVVEDDEDEDGDDLLHHHHGSHCSSSGDPAEYNLRSRTVLCGTCGQPADKASASGSGAQVGG
PISSGSSASSVTVTRSYRSVGGSGGGSFGDNLVTRSYLLGNSSPRTQSPQNCSIIQEMGMRWEVEEGRRKVS
LSCLP

**SEQ ID NO: 7- Lamin A wild-type isoform 5 amino acid**

MDLEAWDPHLEPDAEAMVDGNTKKEGDLIAAQARLKDLEALLNSKEAALSTALSEKRTLEGELHDLRGQVA
KLEAALGEAKKQLQDEMLRRVDAENRLQTMKEELDFQKNIYSEELRETKRRHETRLVEIDNGKQREFESRLA
DALQELRAQHEDQVEQYKKELEKTYSAKLDNARQSAERNSNLVGAAHEELQQSRIRIDSLSAQLSQLQKQL
AAKEAKLRDLEDSLARERDTSRRLLAEKEREMAEMRARMQQQLDEYQELLDIKLALDMEIHAYRKLLEGEEE
RLRLSPSPTSQRSRGRASSHSSQTQGGGSVTKKRKLESTESRSSFSQHARTSGRVAVEEVDEEGKFVRLRNKS
NEDQSMGNWQIKRQNGDDPLLTYRFPPKFTLKAGQVVTIWAAGAGATHSPPTDLVWKAQNTWGCGNS
LRTALINSTGEEVAMRKLVRSVTVVEDDEDEDGDDLLHHHHGSHCSSSGDPAEYNLRSRTVLCGTCGQPA
DKASASGSGAQVGGPISSGSSASSVTVTRSYRSVGGSGGGSFGDNLVTRSYLLGNSSPRTQSPQNCSIM

**SEQ ID NO: 8 - Lamin A wild-type isoform 6 amino acid**

METPSQRRATRSGAQASSTPLSPTRITRLQEKEDLQELNDRLAVYIDRVRSLETENAGLRLRITESEEVVSREVS
GIKAAYEAELGDARKTLDSVAKERARLQLELSKVREEFKELKARNTKKEGDLIAAQARLKDLEALLNSKEAALS
TALSEKRTLEGELHDLRGQVAKLEAALGEAKKQLQDEMLRRVDAENRLQTMKEELDFQKNIYSEELRETKRR
HETRLVEIDNGKQREFESRLADALQELRAQHEDQVEQYKKELEKTYSAKLDNARQSAERNSNLVGAAHEEL
QQSRIRIDSLSAQLSQLQKQLAAKEAKLRDLEDSLARERDTSRRLLAEKEREMAEMRARMQQQLDEYQELL
DIKLALDMEIHAYRKLLEGEEERLRLSPSPTSQRSRGRASSHSSQTQGGGSVTKKRKLESTESRSSFSQHARTS
GRVAVEEVDEEGKFVRLRNKSNEDQSMGNWQIKRQNGDDPLLTYRFPPKFTLKAGQVVTIWAAGAGATH
SPPTDLVWKAQNTWGCGNSLRTALINSTGEEVAMRKLVRSVTVVEDDEDEDGDDLLHHHHGSHCSSSGD
PAEYNLRSRTVLCGTCGQPADKASASGSGAQSPQNCSIM

**SEQ ID NO: 9**

tctgtgtccttcctccaacccttccaggagctgcgtgagaccaagcgacgtcatgagacccgactggtggagattgacaatgggaagca
gcgtgagtttgagagccggctggc

**SEQ ID NO: 10**
ccaccagtcgggtctcagga

**SEQ ID NO: 11**
gtcagatccgctagcgctaccatggtgagcaagggc

**SEQ ID NO: 12**
gtctccatgctagccggtgcag

**SEQ ID NO: 13**
ggctagcatggagaccccgtccc

**SEQ ID NO: 14**
tatagggcgaattgggtaccttacatgatgctgcagttctggggg

**SEQ ID NO: 15**
cgtgagaccaagcgccgtcatgagacccga

**SEQ ID NO: 16**
cgtgagaccaagcgacgtcatgagacccg a

**SEQ ID NO: 17**
gtcatgagaccc

**SEQ ID NO: 18**
gtcattgagaccc

**SEQ ID NO: 19 - LMNA-2A-GFP plasmid vector**

cattcgccattcaggctgcaaataagcgttgatattcagtcaattacaaacattaataacgaagagatgacagaaaaattttcattctgtga
cagagaaaaagtagccgaagatgacggtttgtcacatggagttggcaggatgtttgattaaaaacataacaggaagaaaaatgcccc
gctgtgggcggacaaaatagttgggaactgggaggggtggaaatggagttttttaaggattatttagggaagagtgacaaaatagatg
ggaactgggtgtagcgtcgtaagctaatacgaaaattaaaaatgacaaaatagtttggaactagatttcacttatctggttcggatctccta
ggcgatatcagtgatcagatccagacatgataagatacattgatgagtttggacaaaccacaactagaatgcagtgaaaaaaatgcttt
atttgtgaaatttgtgatgctattgctttatttgtaaccattataagctgcaataaacaagttaacttacttgtacagctcgtccatgccgagag
tgatcccggcggcggtcacgaactccagcaggaccatgtgatcgcgcttctcgttggggtctttgctcagggcggactgggtgctcag
gtagtggttgtcggcagcagcacggggccgtcgccgatgggggtgttctgctggtagtggtcggcgagctgcacgctgccgtcctc
gatgttgtggcggatcttgaagttcaccttgatgccgttcttctgcttgtcggccatgatatagacgttgtggctgttgtagttgtactccagc
ttgtgccccaggatgttgccgtcctccttgaagtcgatgcccttcagctcgatgcggttcaccagggtgtcgccctcgaacttcacctcggc
gcgggtcttgtagttgccgtcgtccttgaagaagatggtgcgctcctggacgtagccttcgggcatggcggacttgaagaagtcgtgct
gcttcatgtggtcggggtagcggctgaagcactgcacgccgtaggtcagggtggtcacgagggtgggccagggcacgggcagctt
gccggtggtgcagatgaacttcagggtcagcttgccgtaggtggcatcgccctcgccctcgccggacacgctgaacttgtggccgttta

EP 4 198 053 A1

cgtcgccgtccagctcgaccaggatgggcaccaccccggtgaacagctcctcgcccttgctcaccatgctagcgtttaaattgtgtaattt
atgtagctgtaattttacccttattaatatttttttacgctttgcattcgacgactgaactcccaaatatatgtttaactcgtcttggtcgtttgaattt
ttgttgctgtgtttcctaatattttccatcaccttaaatatgttattgtaatcctcaatgttgaacttgcaattggacacggcatagttttccatagt
cgtgtaaaacatggtattggctgcattgtaatacatccgactgagcgggtacggatctatgtgtttgagcagcctgttcaaaaactctgcat
cgtcgcaaaacggaatttaacaacaacaattgcattcattttatgtttcaggttcaggggggaggtgtgggaggtttttttaaagcaagtaaa
acctctacaaatgtggtatggctgattatgatcctctagtacttctcgacaagcttacagcttgcatgcctgcaggcagctgcgcgctcgct
cgctcactgaggccgcccgggcaaagcccgggcgtcgggcgacctttggtcgcccggcctcagtgagcgagcgagcgcgcagaga
gggagtggccaactccatcactagggggttccttgtagttaatgattaacccgccatgctacttatctacgtagccatgctctagagcggcc
gcacgcgttcatagcccatatatggagttccgcgttacataacttacggtaaatggcccgcctggctgaccgcccaacgacccccgccca
ttgacgtcaataatgacgtatgttcccatagtaacgccaataggggactttccattgacgtcaatgggtggagtatttacggtaaactgccca
cttggcagtacatcaagtgtatcatatgccaagtacgccccctattgacgtcaatgacggtaaatggcccgcctggcattatgcccagtac
atgaccttatgggactttcctacttggcagtacatctacgtattagtcatcgctattaccatggtgatgcggttttggcagtacatcaatggg
cgtggatagcggtttgactcacggggatttccaagtctccaccccattgacgtcaatgggagtttgttttggcaccaaaatcaacgggact
ttccaaaatgtcgtaacaactccgccccattgacgcaaatgggcggtaggcgtgtacggtgggaggtctatataagcagagctggttta
gtgaaccgtcagatccgctagcgctaccatggtgagcaagggcgaggagctgttcaccggggtggtgcccatcctggtcgagctgga
cggcgacgtaaacggccacaagttcagcgtgtccggcgagggcgagggcgatgccacctacggcaagctgaccctgaagttcatct
gcaccaccggcaagctgcccgtgccctggcccaccctcgtgaccaccctgacctacggcgtgcagtgcttcagccgctaccccgaccac
atgaagcagcacgacttcttcaagtccgccatgcccgaaggctacgtccaggagcgcaccatcttcttcaaggacgacggcaactacaa
gacccgcgccgaggtgaagttcgagggcgacaccctggtgaaccgcatcgagctgaagggcatcgacttcaaggaggacggcaac
atcctggggcacaagctggagtacaactacaacagccacaacgtctatatcatggccgacaagcagaagaacggcatcaaggtgaac
ttcaagatccgccacaacatcgaggacggcagcgtgcagctcgccgaccactaccagcagaacacccccatcggcgacggccccgtg
ctgctgcccgacaaccactacctgagcacccagtccgccctgagcaaagaccccaacgagaagcgcgatcacatggtcctgctggagt
tcgtgaccgccgccgggatcactctcggcatggacgagctgtacaagtactcagatctcgagctcaaggagggcagaggaagtcttct
aacatgcggtgacgtggaggagaatcccggccctgcaccggctagcatggagaccccgtcccagcggcgcgccacccgcagcggg
gcgcaggccagctccactccgctgtcgcccacccgcatcacccggctgcaggagaaggaggacctgcaggagctcaatgatcgcttg
gcggtctacatcgaccgtgtgcgctcgctggaaacggagaacgcagggctgcgccttcgcatcaccgagtctgaagaggtggtcagc
cgcgaggtgtccggcatcaaggccgcctacgaggccgagctcggggatgcccgcaagacccttgactcagtagccaaggagcgcg
cccgcctgcagctggagctgagcaaagtgcgtgaggagtttaaggagctgaaagcgcgcaataccaagaaggagggtgacctgat
agctgctcaggctcggctgaaggacctggaggctctgctgaactccaaggaggccgcactgagcactgctctcagtgagaagcgcac
gctggagggcgagctgcatgatctgcggggccaggtggccaagcttgaggcagccctaggtgaggccaagaagcaacttcaggat
gagatgctgcggcgggtggatgctgagaacaggctgcagaccatgaaggaggaactggacttccagaagaacatctacagtgagg
agctgcgtgagaccaagcgccgtcatgagacccgactggtggagattgacaatgggaagcagcgtgagtttgagagccggctggc
ggatgcgctgcaggaactgcgggcccagcatgaggaccaggtggagcagtataagaaggagctggagaagacttattctgccaag
ctggacaatgccaggcagtctgctgagaggaacagcaacctggtggggggctgcccacgaggagctgcagcagtcgcgcatccgcat
cgacagcctctctgcccagctcagccagctccagaagcagctggcagccaaggaggcgaagcttcgagacctggaggactcactgg
cccgtgagcgggacaccagccggcggctgctggcggaaaaggagcgggagatggccgagatgcgggcaaggatgcagcagca
gctggacgagtaccaggagcttctggacatcaagctggccctggacatggagatccacgcctaccgcaagctcttggagggcgagg
aggagaggctacgcctgtcccccagccctacctcgcagcgcagccgtggccgtgcttcctctcactcatcccagacacagggtggggg
cagcgtcaccaaaaagcgcaaactggagtccactgagagccgcagcagcttctcacagcacgcacgcactagcgggcgcgtggccg

25

tggaggaggtggatgaggagggcaagtttgtccggctgcgcaacaagtccaatgaggaccagtccatgggcaattggcagatcaag

cgccagaatggagatgatcccttgctgacttaccggttcccaccaaagttcaccctgaaggctgggcaggtggtgacgatctgggctgc

aggagctggggccacccacagcccccctaccgacctggtgtggaaggcacagaacacctggggctgcgggaacagcctgcgtacg

gctctcatcaactccactggggaagaagtggccatgcgcaagctggtgcgctcagtgactgtggttgaggacgacgaggatgaggat

ggagatgacctgctccatcaccaccacggctcccactgcagcagctcggggggacccctgctgagtacaacctgcgctcgcgcaccgtgc

tgtgcgggacctgcgggcagcctgccgacaaggcatctgccagcggctcaggagcccaggtgggcggacccatctcctctggctctt

ctgcctccagtgtcacggtcactcgcagctaccgcagtgtggggggcagtgggggtggcagcttcggggacaatctggtcacccgct

cctacctcctgggcaactccagcccccgaacccagagccccagaactgcagcatcatgtaaggtacccaattcgccctatagtgagtc

gtattacgcgcgcagcggccgaccatggcccaacttgtttattgcagcttataatggttacaaataaagcaatagcatcacaaatttcaca

aataaagcattttttttcactgcattctagttgtggtttgtccaaactcatcaatgtatcttatcatgtctggatctccggaccacgtgcggaccg

agcggccgctctagagcatggctacgtagataagtagcatggcgggttaatcattaactacaaggaacccctagtgatggagttggcc

actccctctctgcgcgctcgctcgctcactgaggccgggcgaccaaaggtcgcccgacgcccgggctttgcccgggcggcctcagtga

gcgagcgagcgcgcagctgcctgcaggtctgagacaataaccctgataaatgcttcaataatgtagccaaccactagaactatagctag

agtcctgggcgaacaaacgatgctcgccttccagaaaaccgaggatgcgaaccacttcatccggggtcagcaccaccggcaagcgcc

gcgacggccgaggtcttccgatcctgaagccagggcagatccgtgcacagcaccttgccgtagaagaacagcaaggccgccaat

gcctgacgatgcgtggagaccgaaaccttgcgctcgttcgccagccaggacagaaatgcctcgacttcgctgctgcccaaggttgccg

ggtgacgcacaccgtggaaacggatgaaggcacgaacccagttgacataagcctgttcggttcgtaaactgtaatgcaagtagcgtat

gcgctcacgcaactggtccagaaccttgaccgaacgcagcggtggtaacggcgcagtggcggttttcatggcttgttatgactgtttttttt

gtacagtctatgcctcgggcatccaagcagcaagcgcgttacgccgtgggtcgatgtttgatgttatggagcagcaacgatgttacgca

gcagcaacgatgttacgcagcagggcagtcgccctaaaacaaagttaggtggctcaagtatgggcatcattcgcacatgtaggctcgg

ccctgaccaagtcaaatccatgcgggctgctcttgatcttttcggtcgtgagttcggagacgtagccacctactcccaacatcagccggac

tccgattacctcgggaacttgctccgtagtaagacattcatcgcgcttgctgccttcgaccaagaagcggttgttggcgctctcgcggctta

cgttctgcccaggtttgagcagccgcgtagtgagatctatatctatgatctcgcagtctccggcgagcaccggaggcagggcattgcca

ccgcgctcatcaatctcctcaagcatgaggccaacgcgcttggtgcttatgtgatctacgtgcaagcagattacggtgacgatcccgcag

tggctctctatacaaagttgggcatacgggaagaagtgatgcactttgatatcgacccaagtaccgccacctaacaattcgttcaagccg

agatcggcttcccggccgcggagttgttcggtaaattgtcacaacgccgcgaatatagtctttaccatgcccttggccacgcccctctttaa

tacgacgggcaatttgcacttcagaaaatgaagagtttgctttagccataacaaaagtccagtatgcttttttcacagcataactggactgat

ttcagtttacaactattctgtctagtttaagactttattgtcatagtttagatctattttgttcagtttaagactttattgtccgcccacacccgctta

cgcagggcatccatttattactcaaccgtaaccgattttgccaggttacgcggctggtctgcggtgtgaaataccgcacagatgcgtaag

gagaaaataccgcatcaggcgctcttccgcttcctcgctcactgactcgctgcgctcggtcgttcggctgcggcgagcggtatcagctca

ctcaaaggcggtaatacggttatccacagaatcaggggataacgcaggaaagaacatgtgagcaaaaggccagcaaaaggccagg

aaccgtaaaaaggccgcgttgctggcgttttttccataggctccgcccccctgacgagcatcacaaaaatcgacgctcaagtcagaggtg

gcgaaacccgacaggactataaagataccaggcgtttccccctggaagctccctcgtgcgctctcctgttccgaccctgccgcttaccgg

atacctgtccgcctttctcccttcgggaagcgtggcgctttctcaatgctcacgctgtaggtatctcagttcggtgtaggtcgttcgctccaa

gctgggctgtgtgcacgaaccccccgttcagcccgaccgctgcgccttatccggtaactatcgtcttgagtccaacccggtaagacacga

cttatcgccactggcagcagccactggtaacaggattagcagagcgaggtatgtaggcggtgctacagagttcttgaagtggtggcct

aactacggctacactagaaggacagtatttggtatctgcgctctgctgaagccagttaccttcggaaaaagagttggtagctcttgatccg

gcaaacaaaccaccgctggtagcggtggtttttttgtttgcaagcagcagattacgcgcagaaaaaaaggatctcaagaagatcctttg

atcttttctacggggtctgacgctcagtggaacgaaaactcacgttaagggattttggtcatgagattatcaaaaaggatcttcacctagat

ccttttaaattaaaaatgaagttttaaatcaatctaaagtatatatgagtaaacttggtctgacagttaccaatgcttaatcagtgaggcacct atctcagcgatctgtctatttcgttcatccatagttgcctgactccccgtcgtgtagataactacgatacgggagggcttaccatctggcccc agtgctgcaatgataccgcgagacccacgctcaccggctccagatttatcagcaataaaccagccagccggaagggccgagcgcaga agtggtcctgcaactttatccgcctccatccagtctattaattgttgccgggaagctagagtaagtagttcgccagttaatagtttgcgcaac gttgttgccattgctacaggcatcgtggtgtcacgctcgtcgtttggtatggcttcattcagctccggttcccaacgatcaaggcgagttac atgatcccccatgttgtgcaaaaaagcggttagctccttcggtcctccgatcgttgtcagaagtaagttggccgcagtgttatcactcatgg ttatggcagcactgcataattctcttactgtcatgccatccgtaagatgcttttctgtgactggtgagtactcaaccaagtcattctgagaata gtgtatgcggcgaccgagttgctcttgcccggcgtcaatacgggataataccgcgccacatagcagaactttaaaagtgctcatcattgg aaaacgttcttcggggcgaaaactctcaaggatcttaccgctgttgagatccagttcgatgtaacccactcgtgcacccaactgatcttca gcatcttttactttcaccagcgtttctgggtgagcaaaaacaggaaggcaaaatgccgcaaaaaagggaataagggcgacacggaaa tgttgaatactcatactcttcctttttcaatattattgaagcatttatcagggttattgtctcatgagcggatacatatttgaatgtatttagaaaa ataaacaaataggggttccgcgcacatttccccgaaaagtgccacctgaaattgtaaacgttaatattttgttaaaattcgcgttaaattttg ttaaatcagctcatttttaaccaataggccgaaatcggcaaaatcccttataaatcaaaagaatagaccgagatagggttgagtgttgttc cagtttggaacaagagtccactattaaagaacgtggactccaacgtcaaagggcgaaaaaccgtctatcagggcgatggcccactacg tgaaccatcaccctaatcaagttttttggggtcgaggtgccgtaaagcactaaatcggaaccctaaagggagcccccgatttagagcttg acggggaaagccggcgaacgtggcgagaaaggaagggaagaaagcgaaaggagcgggcgctagggcgctggcaagtgtagc ggtcacgctgcgcgtaaccaccacacccgccgcgcttaatgcgccgctacagggcgcgtc

## Claims

1. Nucleic acid molecule for use in the treatment of laminopathy in a subject, comprising a nucleic acid sequence encoding Lamin A.

2. Nucleic acid molecule for use according to claim 1, wherein the nucleic acid molecule further comprises a nucleic acid sequence encoding a promoter.

3. Nucleic acid molecule for use according to claim 2, wherein the promoter is a constitutive promoter or a tissue-specific promoter.

4. Nucleic acid molecule for use according to any of the preceding claims, wherein the Lamin A is wild-type Lamin A.

5. Nucleic acid molecule for use according to any of the preceding claims, wherein the nucleic acid molecule encodes an amino acid sequence that is at least 70% identical to the amino acid sequence according to SEQ ID NO: 1.

6. Nucleic acid molecule for use according to any of the preceding claims, wherein the laminopathy is caused by one or more mutations of the *LMNA* gene.

7. Nucleic acid molecule for use according to claim 6, wherein the one or more mutations of the *LMNA* gene causes haploinsufficiency for Lamin A or wherein the one or more mutations of the *LMNA* gene causes a dominant-negative Lamin A.

8. Nucleic acid molecule for use according to any one of the preceding claims, wherein the laminopathy is a laminopathy that affects striated muscle, preferably wherein the laminopathy is selected from the group consisting of cardiomyopathy or muscular dystrophy.

9. Nucleic acid molecule for use according to claim 8, wherein the laminopathy is a cardiomyopathy, preferably dilated cardiomyopathy 1A.

10. Vector comprising the nucleic acid molecule of any one of claims 1 to 9 for use in the treatment of laminopathy in a subject.

11. Vector for use according to claim 10, wherein the vector is an expression vector comprising a nucleic acid molecule further comprising a nucleic acid sequence encoding a promoter, wherein the promoter is operably linked to the nucleic acid sequence encoding Lamin A, resulting in expression of Lamin A in cells contacted with the vector when the promoter is active.

12. Vector for use according to any one of claims 10 and 11, wherein the expression of Lamin A from the vector increases the overall expression level of Lamin A in cells affected by laminopathy contacted with the vector to at least 50% of the wild-type expression level, preferably wherein the expression level of Lamin A increases to between about 60% and about 300% of the wild-type expression level.

13. Vector for use according to any one of claims 10 to 12, wherein the expression of Lamin A from the vector increases the absolute force produced by cardiomyocytes affected by laminopathy contacted with the vector by 5% to 200% compared to cardiomyocytes affected by laminopathy not contacted with the vector.

14. AAV for use in the treatment of laminopathy in a subject comprising the nucleic acid molecule for use of any one of claims 1 to 9 or the vector for use of any one of claims 10 to 13, preferably wherein the AAV is serotype AAV6 or AAV9.

15. Pharmaceutical composition for use in the treatment of laminopathy in a subject comprising the nucleic acid molecule for use of any one of claims 1 to 9, the vector for use of any one of claims 10 to 13 or the AAV for use of claim 14, and a pharmaceutically acceptable carrier.

**Figure 1**

**Figure 2A**

**Figure 2B**

**Figure 2C**

C Force Day 19/20

**Figure 2D**

D   Force Day 28/31

**Figure 2E**

E     Last Day of Culture Normalized

**Figure 3**

**Figure 4**

BPM Day 19/20/21

**Figure 5A**

**Figure 5B**

**Figure 5C**

**Figure 5D**

**Figure 6A,B**

**Figure 6C, D**

**Figure 6E**

## Figure 6F

**Figure 7A**

**Figure 7B**

**Figure 8**

## EUROPEAN SEARCH REPORT

Europäisches Patentamt
European Patent Office
Office européen des brevets

**Application Number**

EP 21 21 4769

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/176896 A1 (ENCODED THERAPEUTICS INC [US]) 3 September 2020 (2020-09-03) * [5]-[6], [70], [72], [76], [78]-[79], [8], [10], [48], [124], [127], [141], [145], [13], [16], [33]-[34], [194]-[196], [199]-[201], [104], [90]; claims 49, 57, 60, 66, 69-70, 77; example 1; sequence 12 * | 1-15 | INV. C07K14/78 A61K38/39 A61P43/00 A61P9/00 C12N15/864 C12N15/86 |
| X | AZIBANI FERIEL ET AL: "Gene Therapy via Trans-Splicing for LMNA-Related Congenital Muscular Dystrophy", MOLECULAR THERAPY-NUCLEIC ACIDS, vol. 10, 1 March 2018 (2018-03-01), pages 376-386, XP055925649, US ISSN: 2162-2531, DOI: 10.1016/j.omtn.2017.12.012 Retrieved from the Internet: URL:https://www.ncbi.nlm.nih.gov/pmc/articles/PMC5862133/pdf/main.pdf> * abstract; figure 1 * * p. 376 col. 2, last , p. 379, col. 2, penultimate , p. 383 col. 1, 2nd * | 1-3,6-8, 10,11, 14,15 | |
| Y | H. SAGELIUS ET AL: "Targeted transgenic expression of the mutation causing Hutchinson-Gilford progeria syndrome leads to proliferative and degenerative epidermal disease", JOURNAL OF CELL SCIENCE, vol. 121, no. 7, 11 March 2008 (2008-03-11), pages 969-978, XP055735244, Cambridge ISSN: 0021-9533, DOI: 10.1242/jcs.022913 * the whole document * | 1-15 | TECHNICAL FIELDS SEARCHED (IPC) C07K A61K A61P C12N |

-/--

The present search report has been drawn up for all claims

| Place of search | Date of completion of the search | Examiner |
|---|---|---|
| Munich | 3 June 2022 | Landré, Julien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 21 21 4769

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PEROVANOVIC JELENA ET AL: "Laminopathies disrupt epigenomic developmental programs and cell fate", SCIENCE TRANSLATIONAL MEDICINE, vol. 8, no. 335, 20 April 2016 (2016-04-20), XP055926235, ISSN: 1946-6234, DOI: 10.1126/scitranslmed.aad4991 * the whole document * | 1-15 | |

TECHNICAL FIELDS
SEARCHED       (IPC)

The present search report has been drawn up for all claims

| Place of search | Date of completion of the search | Examiner |
|---|---|---|
| Munich | 3 June 2022 | Landré, Julien |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 21 4769

This annex lists the patent family members relating to the patent documents cited in the above-mentioned European search report.
The members are as contained in the European Patent Office EDP file on
The European Patent Office is in no way liable for these particulars which are merely given for the purpose of information.

03-06-2022

| Patent document cited in search report | Publication date | Patent family member(s) | Publication date |
|---|---|---|---|
| WO 2020176896 A1 | 03-09-2020 | AU 2020229382 A1 | 16-09-2021 |
| | | CA 3131776 A1 | 03-09-2020 |
| | | CN 113840927 A | 24-12-2021 |
| | | CO 2021012310 A2 | 30-09-2021 |
| | | EA 202192376 A1 | 27-12-2021 |
| | | EP 3931336 A1 | 05-01-2022 |
| | | JP 2022522196 A | 14-04-2022 |
| | | KR 20210144696 A | 30-11-2021 |
| | | SG 11202109362X A | 29-09-2021 |
| | | TW 202045725 A | 16-12-2020 |
| | | WO 2020176896 A1 | 03-09-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PALDINO et al.** *Curr Cardiol Rep,* 2018
- **LU et al.** *Dis Model Mech,* 2011
- **MAZZAROTTO et al.** *Circulation,* 2020
- **BECHERT et al.** *Exp Cell Res,* 2003
- **CRASTO et al.** *Front Physiol,* 2018
- **APONTE-UBILLUS et al.** *Appl. Microbiol. Biotechnol.,* 2018
- **ZHONG et al.** *J. Genet. Syndr. Gene Ther.,* 2012
- Remington's Pharmaceutical Sciences. Mack Publishing Co, 1990
- **BRECKWOLDT et al.** *Nat. Protoc.,* 2017
- **MANNHARDT et al.** *Stem Cell Reports,* 2016, vol. 7, 29-42
- **SALEEM et al.** *Stem Cell Reports,* 2020, vol. 14, 312-324
- **BOLGER et al.** *Bioinformatics,* 2014
- **KIM et al.** *Nat Methods,* 2015
- **LI et al.** *Bioinformatics,* 2011
- **ANDERS et al.** *Bioinformatics,* 2015
- **LOVE et al.** *Genome Biol,* 2014
- **GOSWAMI et al.** *Front. Oncol.,* 2019
- **SUBRAMANIAN et al.** *Proc. Natl. Acad. Sci.,* 2005